(19) **Europäisches Patentamt**
**European Patent Office**
**Office européen des brevets**

(11) **EP 4 011 424 B1**

(12) **EUROPÄISCHE PATENTSCHRIFT**

(45) Veröffentlichungstag und Bekanntmachung des Hinweises auf die Patenterteilung:
**01.01.2025 Patentblatt 2025/01**

(21) Anmeldenummer: **21020592.8**

(22) Anmeldetag: **24.11.2021**

(51) Internationale Patentklassifikation (IPC):
**A61M 16/00** *(2006.01)* **A61M 16/01** *(2006.01)*
**A61B 5/08** *(2006.01)* **A61B 5/087** *(2006.01)*

(52) Gemeinsame Patentklassifikation (CPC):
(C-Sets verfügbar)
**A61B 5/0536; A61B 5/0809; A61B 5/0871;**
**A61M 16/024; G16H 20/40; A61M 2230/46** (Forts.)

(54) **SYSTEM ZUR BEATMUNG VON LEBEWESEN**

SYSTEM FOR VENTILATING ORGANISMS

SYSTÈME DE RESPIRATION POUR ORGANISMES VIVANTS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.12.2020 DE 102020007506**
**08.12.2020 DE 102020007492**

(43) Veröffentlichungstag der Anmeldung:
**15.06.2022 Patentblatt 2022/24**

(73) Patentinhaber: **Löwenstein Medical Technology S.A.**
**2557 Luxembourg (LU)**

(72) Erfinder:
• **KREMEIER, Peter**
**76149 Karlsruhe (DE)**
• **WOLL, Christian**
**76470 Ötigheim (DE)**

(74) Vertreter: **Löwenstein Medical IP**
**Löwenstein Medical Technology**
**GmbH + Co.KG IP Management**
**Kronsaalsweg 40**
**22525 Hamburg (DE)**

(56) Entgegenhaltungen:
DE-A1- 102016 011 161  DE-A1- 102017 007 224
DE-A1- 102020 002 279  DE-B3- 102015 006 902

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)

C-Sets
A61M 2230/46, A61M 2230/005

## Beschreibung

Die Erfindung betrifft ein System zur Beatmung von Lebewesens.

Die EIT (Elektro-Impedanz-Tomographie) lässt es zu, räumlich differenzierte Informationen zum Zustand einer Lunge zu ermitteln. Die EIT ist ein nicht-invasives Verfahren, bei dem schwache Wechselströme durch den Körper fließen und dabei elektrische Spannungen an der Oberfläche erzeugt werden. Bei der EIT werden die Oberflächenspannungen oder Leitfähigkeiten am Körper gemessen, welche von den Impedanzen beziehungsweise deren Verteilung innerhalb beispielsweise des Thorax abhängen. Dabei werden Pixel aufgenommen, dabei gibt jedes Pixel eine elektrische Leitfähigkeit beziehungsweise Impedanz wieder. Die gemessene Impedanz wird zum überwiegenden Teil durch den intrapulmonalen Luftgehalt bestimmt, es lassen sich so also auch Rückschlüsse auf den Zustand der Lungenbläschen in bestimmten Bereichen der Lunge ziehen. Aufgrund der hohen zeitlichen Auflösung bietet dieses Verfahren die Möglichkeit, schnelle physiologische Veränderungen zu erfassen. Die Auswertung der EIT-Messsignale und die Umwandlung in Bilder ist Gegenstand verschiedener wissenschaftlicher Publikationen (vgl. Lionheart, W.R.B. in Physiological Measurement 2004, 25, 1 und Schullcke, B. in Scientific Reports, 2016, 6, Seite 25951ff). Weitere allgemeine Details lassen sich zum Beispiel auch der DE 10 2013 203177 A1 entnehmen. DE 10 2017 007 224 A1 offenbart eine Vorrichtung und ein Verfahren zur Elektro-Impedanz-Tomographie mit einer Bestimmung von Differenzkennzahlen auf Basis von EIT-Daten.

Darüber hinaus ist es im Stand der Technik bekannt, mit Hilfe von EIT-Messungen passende Einstellungen des positiven end-exspiratorischen Druck (PEEP) zu finden. So zeigten Costa et al. (Costa, E.L. in Intensive Care Med 2009; 35, Seiten 1132-1137) durch eine stufenweise Veränderung des PEEP und gleichzeitiger EIT-Messungen, wie passende Einstellungen des PEEP gefunden werden können. Da diese Methode zeitaufwändig ist, kommt sie insbesondere nur zu Beginn einer Beatmung in Frage. Das Bedürfnis des Lebewesens kann sich über den Zeitraum der Beatmung ändern und insbesondere im Bereich der Medizin bleibt häufig keine ausreichende Zeit um diese Methode häufiger anzuwenden.

Aufgabe der vorliegenden Erfindung ist daher die Bereitstellung eines Systems zur Analyse einer effektiven und sicheren Beatmung eines Lebewesens. Die Aufgabe wird durch das erfinderische System gemäß Anspruch 1 gelöst.

Das System zur Beatmung eines Lebewesens, umfassend zumindest ein Beatmungsgerät und zumindest eine EIT-Messeinrichtung, wobei das Beatmungsgerät zumindest eine steuerbare Atemgasquelle sowie eine programmierbare Steuereinheit zur Steuerung der Atemgasquelle umfasst und wobei die EIT-Messeinrichtung zumindest eine Sensorvorrichtung zur Messung von Impedanzwerten zumindest einer Lunge des Lebewesens sowie zumindest eine Berechnungs- und Auswerteeinheit umfasst, wobei das System die über die zumindest eine Sensorvorrichtung gemessenen Impedanzwerte Pixeln n zuordnet, wobei die Steuereinheit dazu eingerichtet ist,

> a. in einer Referenzphase R der Atemgasquelle Beatmungseinstellungen vorzugeben, wobei zumindest einem Zeitpunkt der Referenzphase Impedanzwerte aufgenommen werden und aus den aufgenommenen Impedanzwerten für jedes Pixel n eine erste Kennzahl $C_{R,n}$ berechnet wird und
> b. in zumindest einer an die Referenzphase R anschließenden Testphase T1 der Atemgasquelle Beatmungseinstellungen vorzugeben, wobei sich die Beatmungseinstellungen in zumindest einer Beatmungseinstellung von den Beatmungseinstellungen der Referenzphase unterscheiden und wobei zu zumindest einem Zeitpunkt der Testphase T1 Impedanzwerte aufgenommen werden und aus den aufgenommenen Impedanzwerten für jedes Pixel n eine zweite Kennzahl $C_{T1,n}$ berechnet wird.

In manchen Ausführungsformen des Systems schließt an die zumindest eine Testphase T1 zumindest eine weitere Testphase Tm an, wobei die Beatmungseinstellung jeder weiteren Testphase Tm sich von der ersten Testphase T1 und der Referenzphase R in zumindest einer Beatmungseinstellung unterscheidet und wobei das System zu zumindest einem Zeitpunkt jeder weiteren Testphase Tm Impedanzwerte aufnimmt und aus den aufgenommenen Impedanzwerten für jedes Pixel n eine zweite Kennzahl $C_{Tm,n}$ berechnet.

In manchen Ausführungsformen folgt des Systems auf die Referenzphase R nur eine Testphase.

Erfindungsgemäß umfasst sowohl die Referenzphase als auch jede Testphase zumindest zwei Atemzüge, wobei die Referenzphase und jede Testphase in zumindest zwei Unterphasen unterteilt werden können, wobei zumindest eine Unterphase eine Gewöhnungsphase darstellt und zumindest eine Unterphase eine Messphase darstellt.

In manchen Ausführungsformen des Systems wird bei jedem Atemzug während der Exspirationsphase ein positiver end-exspiratorischer Druck (PEEP) appliziert und während der Inspirationsphase wird ein um den Wert ΔP höherer inspiratorischer Druck $P_{insp}$ oder um einen vorgegebenen Faktor höheres Tidalvolumen VT appliziert.

In manchen Ausführungsformen des Systems wird zum Ende zumindest einer Exspirationsphase ein endexspiratorisches Hold-Manöver durchgeführt, wobei das endexspiratorische Hold-Manöver ein Halten des PEEP und bedarfsweisen die Ermittlung des intrinsischen PEEP (PEEPi) umfasst.

In manchen Ausführungsformen des Systems wird zum Ende zumindest einer Inspirationsphase ein endinspiratorisches Hold-Manöver durchgeführt, wobei das endinspiratorische Hold-Manöver ein Halten des inspiratorischen Druckes $P_{insp}$ und bedarfsweisen die Ermittlung des Plateaudrucks und/oder ein Surrogat für eine statische Compliance umfasst.

In manchen Ausführungsformen des Systems werden das endexspiratorische Hold-Manöver und das endinspiratorische Hold-Manöver während der Messphase durchgeführt.

In manchen Ausführungsformen des Systems umfasst die Messphase zumindest zwei Atemzüge, wobei während der Exspirationsphase des vorletzten Atemzugs das endexspiratorische Hold-Manöver durchgeführt wird und wobei während der Inspirationsphase des letzten Atemzuges das endinspiratorische Hold-Manöver durchgeführt wird.

In manchen Ausführungsformen des Systems werden sowohl während der Referenzphase R als auch während der Testphase T1 zu zumindest zwei Zeitpunkten Impedanzwerte aufgenommen und die Impedanzwerte Pixeln n zugeordnet, wobei der erste Zeitpunkt während des endexspiratorischen Hold-Manövers liegt und die aufgenommenen Impedanzwerte den Pixeln n als $I_{Min,n}$ zugeordnet werden und wobei der zweite Zeitpunkt während des endinspiratorischen Hold-Manövers liegt und die aufgenommenen Impedanzwerteden Pixeln n als $I_{Max,n}$ zugeordnet werden.

In manchen Ausführungsformen des Systems werden jeweils für die Referenzphase und Testphase aus den jeweiligen Werten $I_{Min,n}$ und $I_{Max,n}$ folgende Werte berechnet:

a. $\Delta I_n$ für jedes Pixel n als Differenz aus $I_{Max,n}$ und $I_{Min,n}$,
b. $I_{min,Sum}$ als Summe aller $I_{Min,n}$,
c. $I_{Max,Sum}$ als Summer aller $I_{Max,n}$,
d. $\Delta I_{global}$ als Differenz von $I_{Max,Sum}$ und $I_{Min,Sum}$,

wobei die Berechnung für das jeweilige Pixel nur dann durchgeführt wird und das Pixel nur dann in die Summen $I_{Max,Sum}$ und $I_{Min,Sum}$ einbezogen wird, wenn für das Pixel gilt $\Delta I_n > 0$.

In manchen Ausführungsformen des Systems werden die jeweiligen Kennzahlen $C_{R,n}$ und $C_{T1,n}$ der Referenz- bzw. Testphase jeweils aus dem Verhältnis von $\Delta I_n$ zu $\Delta I_{global}$ berechnet, wobei

a. das Verhältnis $\Delta I_n$ zu $\Delta I_{global}$ der Referenzphase mit einem Faktor $C_{R,Stat}$ multipliziert wird um $C_{R,n}$ zu berechnen und
b. das Verhältnis $\Delta I_n$ zu $\Delta I_{global}$ der Testphase mit einem Faktor $C_{T1,Stat}$ multipliziert wird um $C_{T1,n}$ zu berechnen,

und wobei $C_{R,Stat}$ und $C_{T1,Stat}$ aus der Druckdifferenz des endexspiratorischen Hold-Manövers und des endinspiratorischen Hold-Manövers sowie dem Exspirationsvolumen des Atemzuges, welcher dem Atemzug, währenddessen das endexspiratorische Hold-Manöver durchgeführt wird, vorrausgeht, bestimmt werden.

In manchen Ausführungsformen des Systems wird die Kennzahl $C_{T1,n}$ jedes Pixels n mit der Kennzahl $C_{R,n}$ des gleichen Pixels n verglichen und das Pixel n einer von zumindest zwei Zahlengruppen zugeordnet.

In manchen Ausführungsformen des Systems werden

a. die Pixel n für die gilt, dass $C_{T1,n}$ größer ist als $C_{R,n}$ einer ersten Zahlengruppe WIN zugeordnet und
b. die Pixel n, für die gilt, dass $C_{T1,n}$ kleiner ist als $C_{R,n}$ einer zweiten Zahlengruppe LOSS zugeordnet.

In manchen Ausführungsformen des Systems wird für jedes Pixel n der jeweiligen Zahlengruppe aus den Kennzahlen $C_{T1,n}$ und $C_{R,n}$ eine Kennzahl berechnet, wobei

a. für die Pixel n aus der Zahlengruppe WIN eine Kennzahl $C_{WIN,n}$ berechnet wird und
b. für die Pixel n aus der Zahlengruppe LOSS eine Kennzahl $C_{LOSS,n}$ berechnet wird.

In manchen Ausführungsformen des Systems wird für jede Zahlengruppe WIN und LOSS mit der Summe der jeweiligen Kennzahlen $C_{WIN,n}$ beziehungsweise $C_{LOSS,n}$ jeweils eine globale Kennzahl berechnet, wobei

a. für die Zahlengruppe WIN mit der Summe der Kennzahlen $C_{WIN,n}$ eine Kennzahl $C_{WIN,global}$ berechnet wird und
b. für die Zahlengruppe LOSS mit der Summe der Kennzahlen $C_{LOSS,n}$ eine Kennzahl $C_{LOSS,global}$ berechnet wird.

In manchen Ausführungsformen des Systems werden die Kennzahlen $C_{WIN,global}$ und $C_{LOSS,global}$ interpretiert und die Größe der Kennzahlen alphanumerisch und/oder graphisch ausgegeben.

In manchen Ausführungsformen des Systems werden bei der Interpretation der Kennzahlen die Beatmungseinstellung einbezogen, in welcher sich die Referenzphase und die Testphase T1 unterscheiden.

In manchen Ausführungsformen des Systems wird anhand der Interpretation der Kennzahlen $C_{WIN,global}$ und $C_{LOSS,global}$ eine Meldung generiert, wobei die Meldung zumindest eine Empfehlung bezüglich der Beatmungseinstellungen enthält und wobei die Meldung alphanumerisch und/oder graphisch ausgegeben wird.

In manchen Ausführungsformen des Systems kann als die Beatmungseinstellung, die sich zwischen Referenzphase und Testphase unterscheidet zumindest eines von $\Delta P$ und/oder PEEP und/oder ein Tidalvolumen eingestellt werden.

In manchen Ausführungsformen des Systems wird bei einer PEEP-Einstellung, welche in der Testphase T1 gegenüber der Referenzphase erhöht ist, und

a. wenn $C_{WIN,global}$ und $C_{LOSS,global}$ unter dem eingestellten Schwellenwert liegen, die Empfehlung ausgegeben, die Beatmungseinstellungen nicht zu verändern,

b. wenn $C_{WIN,global}$ über dem eingestellten Schwellenwert liegt und $C_{LOSS,global}$ unter dem eingestellten Schwellenwert liegt, die Empfehlung ausgegeben, den PEEP zu erhöhen,

c. wenn $C_{WIN,global}$ unter dem eingestellten Schwellenwert liegt und $C_{LOSS,global}$ über dem eingestellten Schwellenwert liegt, die Empfehlung ausgegeben, die den PEEP nicht zu verändern und eine Reduzierung vom inspiratorischen Druck $P_{insp}$ und/oder dem exspiratorischen Tidalvolumen VT zu erwägen, wobei zunächst eine Testphase mit reduziertem inspiratorischen Druck $P_{insp}$ eingestellt werden soll,

d. wenn $C_{WIN,global}$ und $C_{LOSS,global}$ über dem eingestellten Schwellenwert liegen, die Empfehlung ausgegeben, den PEEP zu erhöhen und eine Reduzierung vom inspiratorischen Druck $P_{insp}$ und/oder exspiratorischen Tidalvolumen VT zu erwägen, wobei zunächst eine Testphase mit reduziertem inspiratorischen Druck $P_{insp}$ eingestellt werden soll.

In manchen Ausführungsformen des Systems wird bei einer PEEP-Einstellung, welche in der Testphase T1 gegenüber der Referenzphase reduziert ist

a. wenn $C_{WIN,global}$ und $C_{LOSS,global}$ unter dem eingestellten Schwellenwert liegen, die Empfehlung ausgegeben, die Beatmungseinstellungen nicht zu verändern,

b. wenn $C_{WIN,global}$ über dem eingestellten Schwellenwert liegt und $C_{LOSS,global}$ unter dem eingestellten Schwellenwert liegt, die Empfehlung ausgegeben, den PEEP zu reduzieren,

c. wenn $C_{WIN,global}$ unter dem eingestellten Schwellenwert liegt und $C_{LOSS,global}$ über dem eingestellten Schwellenwert liegt, die Empfehlung ausgegeben, die Einstellung des PEEP nicht zu verändern und ein Rekrutierungsmanöver zu erwägen,

d. wenn $C_{WIN,global}$ und $C_{LOSS,global}$ über dem eingestellten Schwellenwert liegen, die Empfehlung ausgegeben, die Einstellung des PEEP nicht zu verändern und ein Rekrutierungsmanöver zu erwägen sowie, soweit möglich, $P_{insp}$ und/oder VT zu reduzieren.

In manchen Ausführungsformen des Systems wird bei einer $P_{insp}$- und/oder VT-Einstellung, welche in der Testphase T1 gegenüber der Referenzphase erhöht ist und

a. wenn $C_{WIN,global}$ und $C_{LOSS,global}$ unter dem eingestellten Schwellenwert liegen, die Empfehlung ausgegeben, die Beatmungseinstellungen nicht zu verändern

b. wenn $C_{WIN,global}$ über dem eingestellten Schwellenwert liegt und $C_{LOSS,global}$ unter dem eingestellten Schwellenwert liegt, die Empfehlung ausgegeben, den PEEP zu erhöhen

c. wenn $C_{WIN,global}$ unter dem eingestellten Schwellenwert liegt und $C_{LOSS,global}$ über dem eingestellten Schwellenwert liegt, die Empfehlung ausgegeben, die Einstellung des PEEP und/oder des $P_{insp}$ und/oder von VT zu reduzieren

d. wenn $C_{WIN,global}$ und $C_{LOSS,global}$ über dem eingestellten Schwellenwert liegen, die Empfehlung ausgegeben, den PEEP zu erhöhen und, soweit möglich, den $P_{insp}$ und/oder das Tidalvolumen zu reduzieren.

In manchen Ausführungsformen des Systems wird bei einer $P_{insp}$- und/oder VT-Einstellung, welche in der Testphase T1 gegenüber der Referenzphase reduziert ist und

a. wenn $C_{WIN,global}$ und $C_{LOSS,global}$ unter dem eingestellten Schwellenwert liegen, die Empfehlung ausgegeben, die Beatmungseinstellungen nicht zu verändern,

b. wenn $C_{WIN,global}$ über dem eingestellten Schwellenwert liegt und $C_{LOSS,global}$ unter dem eingestellten Schwellenwert liegt, die Empfehlung ausgegeben, den PEEP und/oder $P_{insp}$ und/oder VT zu reduzieren,

c. wenn $C_{WIN,global}$ unter dem eingestellten Schwellenwert liegt und $C_{LOSS,global}$ über dem eingestellten Schwellenwert liegt, die Empfehlung ausgegeben, den PEEP zu erhöhen,

d. wenn $C_{WIN,global}$ und $C_{LOSS,global}$ über dem eingestellten Schwellenwert liegen, die Empfehlung ausgegeben, den PEEP zu erhöhen und, soweit möglich, den $P_{insp}$ und/oder das Tidalvolumen zu reduzieren.

In manchen Ausführungsformen des Systems wird anhand der Interpretation der Kennzahlen $C_{WIN,global}$ und $C_{LOSS,global}$ ein Befund erstellt.

In manchen Ausführungsformen des Systems enthält die Meldung neben der Empfehlung auch zumindest einen Befund.

In manchen Ausführungsformen des Systems wird bei einer PEEP-Einstellung, welche in der Testphase (T1) gegenüber der Referenzphase (R) erhöht ist, und

a. wenn $C_{WIN,global}$ und $C_{LOSS,global}$ unter dem eingestellten Schwellenwert liegen, ein Befund erstellt, dass keine signifikanten Änderungen festgestellt wurden,

b. wenn $C_{WIN,global}$ über dem eingestellten Schwellenwert liegt und $C_{LOSS,global}$ unter dem eingestellten Schwellenwert liegt, ein Befund erstellt, dass es zu einer Rekrutierung bei Erhöhung des PEEP kommt,

c. wenn $C_{WIN,global}$ unter dem eingestellten Schwellenwert liegt und $C_{LOSS,global}$ über dem eingestellten Schwellenwert liegt, ein Befund erstellt, dass es durch die Erhöhung des PEEP zu einer Überdehnung kommt,

d. wenn $C_{WIN,global}$ und $C_{LOSS,global}$ über dem eingestellten Schwellenwert liegen, ein Befund erstellt, dass es durch die Erhöhung des PEEP sowohl zu einer Rekrutierung als auch zu einer Überdehnung kommt.

In manchen Ausführungsformen des Systems wird bei einer PEEP-Einstellung, welche in der Testphase (T1) gegenüber der Referenzphase (R) reduziert ist

a. wenn $C_{WIN,global}$ und $C_{LOSS,global}$ unter dem eingestellten Schwellenwert liegen, ein Befund erstellt, dass keine signifikanten Änderungen festgestellt wurden,

b. wenn $C_{WIN,global}$ über dem eingestellten Schwellenwert liegt und $C_{LOSS,global}$ unter dem eingestellten Schwellenwert liegt, ein Befund erstellt, dass eine nachlassende Überdehnung zu beobachten ist,

c. wenn $C_{WIN,global}$ unter dem eingestellten Schwellenwert liegt und $C_{LOSS,global}$ über dem eingestellten Schwellenwert liegt, ein Befund erstellt, dass eine Derekrutierung zu beobachten ist,

d. wenn $C_{WIN,global}$ und $C_{LOSS,global}$ über dem eingestellten Schwellenwert liegen, ein Befund erstellt, dass sowohl eine nachlassende Überdehnung als auch eine Derektutierung zu beobachten ist.

In manchen Ausführungsformen des Systems wird bei einer $P_{insp}$- und/oder VT-Einstellung, welche in der Testphase (T1) gegenüber der Referenzphase (R) erhöht ist und

a. wenn $C_{WIN,global}$ und $C_{LOSS,global}$ unter dem eingestellten Schwellenwert liegen, ein Befund erstellt, dass keine signifikanten Änderungen zu beobachten sind,

b. wenn $C_{WIN,global}$ über dem eingestellten Schwellenwert liegt und $C_{LOSS,global}$ unter dem eingestellten Schwellenwert liegt, ein Befund erstellt, dass der Verdacht einer tidalen Rekrutierung zu beobachten ist,

c. wenn $C_{WIN,global}$ unter dem eingestellten Schwellenwert liegt und $C_{LOSS,global}$ über dem eingestellten Schwellenwert liegt, ein Befund erstellt, dass eine Überdehnung zu beobachten ist,

d. wenn $C_{WIN,global}$ und $C_{LOSS,global}$ über dem eingestellten Schwellenwert liegen, ein Befund erstellt, dass eine Überdehnung zu beobachten ist.

In manchen Ausführungsformen des Systems wird bei einer $P_{insp}$- und/oder VT-Einstellung, welche in der Testphase (T1) gegenüber der Referenzphase (R) reduziert ist und

a. wenn $C_{WIN,global}$ und $C_{LOSS,global}$ unter dem eingestellten Schwellenwert liegen, ein Befund erstellt, dass keine signifikanten Änderungen zu beobachten sind,

b. wenn $C_{WIN,global}$ über dem eingestellten Schwellenwert liegt und $C_{LOSS,global}$ unter dem eingestellten Schwellenwert liegt, ein Befund erstellt, dass eine nachlassende Überdehnung zu beobachten ist,

c. wenn $C_{WIN,global}$ unter dem eingestellten Schwellenwert liegt und $C_{LOSS,global}$ über dem eingestellten Schwellenwert liegt, ein Befund erstellt, dass der Verdacht auf eine tidale Rekrutierung zu beobachten ist,

d. wenn $C_{WIN,global}$ und $C_{LOSS,global}$ über dem eingestellten Schwellenwert liegen, ein Befund erstellt, dass eine nachlassende Überdehnung und ein Verdacht auf eine tidale Rekrutierung zu beobachten ist.

In manchen Ausführungsformen des Systems werden ausgegebenen Empfehlungen automatisch umgesetzt und entsprechende Beatmungseinstellungen vorgenommen.

Die Aufgabe wird ferner gelöst durch ein System zur Beatmung eines Lebewesens, umfassend zumindest ein Beatmungsgerät und zumindest eine EIT-Messeinrichtung, wobei das Beatmungsgerät zumindest eine steuerbare Atemgasquelle sowie eine programmierbare Steuereinheit zur Steuerung der Atemgasquelle umfasst und wobei die EIT-Messeinrichtung zumindest eine Sensorvorrichtung zur Messung von Impedanzwerten zumindest einer Lunge des Lebewesens sowie zumindest eine Berechnungs- und Auswerteeinheit umfasst, wobei das System die über die zumindest eine Sensorvorrichtung gemessenen Impedanzwerte Pixeln n zuordnet, wobei das System dazu eingerichtet ist folgende Verfahrensschritte durchzuführen

a. Gewöhnen des Lebewesens an Beatmungseinstellungen einer Referenzphase R

b. Messen von Impedanzen der Lunge des Lebewesens während der Referenzphase R

c. Gewöhnen des Lebewesens an Beatmungseinstellungen einer Testphase T1

d. Messen von Impedanzen der Lunge des Lebewesens während der Testphase T1

e. Berechnen von Kennzahlen $C_{R,n}$ und $C_{T1,n}$ aus den Impedanzwerten aus dem Schritt des Messens

f. Vergleichen der Kennzahlen $C_{R,n}$ und $C_{T1,n}$

g. Einordnen der Kennzahlen $C_{R,n}$ und $C_{T1,n}$ in Zahlengruppen WIN und LOSS

h. Berechnen von Kennzahlen $C_{WIN,global}$ und $C_{LOSS,global}$

i. Vergleichen der Kennzahlen $C_{WIN,global}$ und $C_{LOSS,global}$ mit Schwellenwerten

j. Auswerten, wobei das Auswerten zumindest die Generierung einer graphischen Darstellung der Kennzahlen $C_{WIN,global}$ und $C_{LOSS,global}$ umfasst und wobei der Schritt des Auswertens (113) optional die Erstellung eines Befundes unter Berücksichtigung der Kennzahlen $C_{WIN,global}$ und $C_{LOSS,global}$ umfasst

k. Empfehlen, wobei das Empfehlen zumindest die Generierung einer alphanumerischen Ausgabe von Empfehlungen bezüglich der Beatmungseinstellungen umfasst.

Die Aufgabe wird ferner gelöst durch ein Verfahren zur Beatmung eines Lebewesens mit System zur Beatmung eines Lebewesens, umfassend zumindest ein Beatmungsgerät und zumindest eine EIT-Messeinrichtung, wobei das Beatmungsgerät zumindest eine steuerbare Atemgasquelle sowie eine programmierbare Steuereinheit zur Steuerung der Atemgasquelle umfasst und wobei die EIT-Messeinrichtung zumindest eine Sensorvorrichtung zur Messung von Impedanzwerten zumindest einer Lunge des Lebewesens sowie zumindest eine Berechnungs- und Auswerteeinheit umfasst, wobei das System die über die zumindest eine Sensorvorrichtung gemessenen Impedanzwerte Pixeln n zuordnet, wobei die Steuereinheit

a. in einer Referenzphase R der Atemgasquelle Beatmungseinstellungen vorgibt, wobei zu zumindest einem Zeitpunkt der Referenzphase Impedanzwerte aufgenommen werden und aus den aufgenommenen Impedanzwerten für jedes Pixel n eine erste Kennzahl $C_{R,n}$ berechnet wird und

b. in zumindest einer an die Referenzphase R anschließenden Testphase T1 der Atemgasquelle Beatmungseinstellungen vorgibt, wobei sich die Beatmungseinstellungen in zumindest einer Beatmungseinstellung von den Beatmungseinstellungen der Referenzphase unterscheiden und wobei zu zumindest einem Zeitpunkt der Testphase T1 Impedanzwerte aufgenommen werden und aus den aufgenommenen Impedanzwerten für jedes Pixel n eine zweite Kennzahl $C_{T1,n}$ berechnet wird.

Es ist darauf hinzuweisen, dass die in den Ansprüchen einzeln aufgeführten Merkmale in beliebiger, technisch sinnvoller Weise miteinander kombiniert werden können und weitere Ausgestaltungen der Erfindung aufzeigen. Die Beschreibung charakterisiert und spezifiziert die Erfindung insbesondere im Zusammenhang mit den Figuren zusätzlich.

Es sei ferner darauf hingewiesen, dass eine hierin verwendete, zwischen zwei Merkmalen stehende und diese miteinander verknüpfende Konjunktion "und/oder" stets so auszulegen ist, dass in einer ersten Ausgestaltung des erfindungsgemäßen Gegenstands lediglich das erste Merkmal vorhanden sein kann, in einer zweiten Ausgestaltung lediglich das zweite Merkmal vorhanden sein kann und in einer dritten Ausgestaltung sowohl das erste als auch das zweite Merkmal vorhanden sein können.

Unter einem Beatmungsgerät ist jedwedes Gerät zu verstehen, welches einen Anwender oder Patienten bei der natürlichen Atmung unterstützt, die Beatmung des Anwenders bzw. Patienten übernimmt und/oder zur Atemtherapie dient und/oder anderweitig die Atmung des Anwenders bzw. Patienten beeinflusst. Darunter fallen zum Beispiel, aber nicht ausschließend, CPAPsowie BiPAP-Geräte, Narkose- bzw. Anästhesiegeräte, Atemtherapiegeräte, (klinische, außerklinische oder Notfall-) Beatmungsgeräte, Highflow-Therapiegeräte und Hustenmaschinen. Beatmungsgeräte können auch als Diagnosegeräte für die Beatmung verstanden werden. Diagnosegeräte können dabei allgemein zur Erfassung von medizinischen Parametern eines Patienten dienen. Darunter fallen auch Geräte, welche medizinische Parameter von Patienten in Kombination mit oder ausschließlich die Atmung betreffend, erfassen und optional verarbeiten können.

Als Patienteninterface kann, soweit nicht ausdrücklich anders beschrieben, jeglicher Teil oder verbundene Peripheriegeräte des Beatmungsgerätes verstanden werden, welche zur Interaktion, insbesondere zu Therapie- oder Diagnosezwecken, mit einem Patienten gedacht ist. Insbesondere kann ein Patienteninterface als Trachealkanüle, als Maske eines Beatmungsgerätes bzw. eine mit dem Beatmungsgerät verbundene Maske verstanden werden. Diese Maske kann eine Full-Face Maske, also Nase und Mund umschließende, oder eine Nasenmaske, also eine nur die Nase umschließende Maske, sein. Auch Trachealtuben und sogenannte Nasenbrillen können als Maske eingesetzt werden. In manchen Fällen kann das Patienteninterface auch ein einfaches Mundstück, beispielsweise ein Rohr, sein, durch welches das Lebewesen zumindest ausatmet.

Die erfinderische Messeinrichtung eignet sich insbesondere nicht nur zur Anwendung im Bereich der Therapie und Beatmung von Patienten, sondern kann darüber hinaus auch Anwendung in anderen Bereichen, bei denen eine Unterstützung der natürlichen Atmung gewünscht sein kann, wie zum Beispiel bei Tauchern, Bergsteigern, in der Schutzausrüstung von Einsatzkräften der Feuerwehr, etc., finden. Auch im Bereich der Bestimmung verschiedener physiologischer Parameter eines Lebewesens - nicht nur in Hinblick einer Diagnostik - lässt sich die erfinderische Messeinrichtung einsetzen.

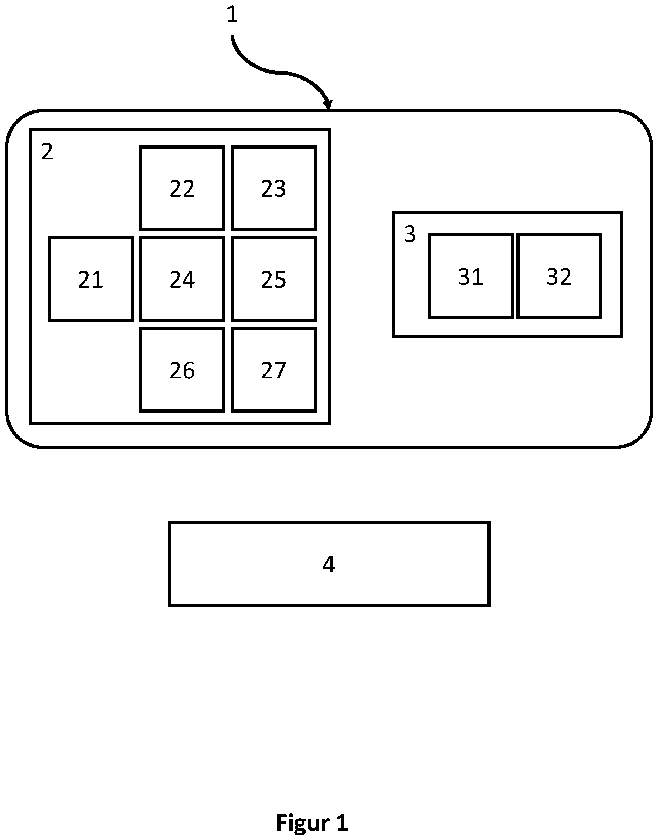

Folgend wird das erfindungsgemäße System anhand der Figuren 1 bis 5 beispielhaft näher beschrieben.

Die folgend beschriebenen Schritte, Berechnungen und Empfehlungen können ausdrücklich zumindest teilweise auch durch etwa Auswerte-, Berechnungs- und/oder Steuereinheiten des Beatmungsgerätes 2 durchgeführt werden. Insbesondere die beispielhaft der EIT-Messeinrichtung 3 zugeordneten Berechnungen, Auswertungen, Interpretationen, Ausgaben und/oder Empfehlungen können in identischer und/oder leicht abgewandelter Weise von dem Beatmungsgerät 2 und/oder allgemein durch das System 1 vollzogen werden. Beispielsweise umfasst das System 1 dazu zumindest eine übergeordnete Steuereinheit sowie gegebenenfalls entsprechende Berechnungs- und Auswerteeinheiten. Insgesamt ist das System (1) vorzugsweise so ausgelegt, dass alle Schritte, Berechnungen, Empfehlungen, Auswertung (inkl. Erstellen von Befunden), Interpretationen und/oder Ausgaben automatisch durchführen kann. In der Regel soll es lediglich notwendig sein, dass die Beatmungseinstellungen für die Referenzphasen und Testphasen manuell eingestellt werden.

Figur 1 zeigt schematisch eine beispielhafte Ausführungsform des Systems 1 bestehend aus einem Beatmungsgerät 2 und einer EIT-Messeinrichtung 3. Das System 1 ist beispielsweise mit einem Lebewesen 4 verbunden, wobei zum einen das Beatmungsgerät 1 zum Beispiel über ein oder mehrere Schläuche und Patienteninterface (beides nicht dargestellt) mit dem Lebewesen verbunden ist um Atemgas zum und ggf. weg vom Lebewesen 4 zu fördern. Das Beatmungsgerät umfasst zumindest eine steuerbare Atemgasquelle 21, eine programmierbare Steuereinheit 22, eine Sensoreinheit 23, eine Speichereinheit 24, eine Aufbereitungseinheit 25, eine Überwachungseinheit 26 sowie eine Erkennungseinheit 27. In der Regel umfasst das Beatmungsgerät 2 zudem auch noch zumindest eine Benutzerschnittstelle (nicht dargestellt), über welche zumindest Eingaben am Beatmungsgerät 2 gemacht werden können. Auch kann das Beatmungsgerät 2 eine Anzeigevorrichtung umfassen oder über eine Schnittstelle an eine Anzeigeeinheit angeschlossen sein, über welche Daten, Werte, Informationen, Meldungen, etc. angezeigt beziehungsweise ausgegeben werden können.

Die steuerbare Atemgasquelle 21 ist beispielsweise dazu ausgebildet, Atemgas zum Lebewesen 4 zu fördern. Eine Atemgasquelle 21 kann beispielsweise eine Gebläse- und/oder Ventileinheit sein, welche aus der Umgebungsluft und/oder aus Gasflaschen das Atemgas generiert.

Die Steuereinheit 22 dient beispielsweise zur Steuerung des Beatmungsgerätes 2, insbesondere der Atemgasquelle 21. Dazu ist die Steuereinheit 22 unter anderem ausgebildet, den durch die Atemgasquelle 21 generierten Beatmungsdruck und/oder -fluss zu steuern. Auch kann die Steuereinheit 22 dazu ausgebildet sein, andere Bestandteile und/oder Einheiten des Beatmungsgerätes 2 zu steuern. In manchen Ausführungsformen kann die Steuereinheit 22 auch weiter unterteilt sein und aus mehreren Steuereinheiten bestehen, welche jeweils eine individuelle Einheit und/oder Bestandteil des Beatmungsgerätes 2 steuern. Insbesondere ist die Steuereinheit 22 dazu eingerichtet, das Beatmungsgerät 2 zumindest teilweise automatisch zu steuern. Insbesondere ist die Steuereinheit 22 so programmierbar, dass verschiedene Manöver, während denen zumindest eine Beatmungseinstellung geändert wird, durchzuführen.

Die Sensoreinheit 23 ist eingerichtet, Messwerte, insbesondere Parameter, die mit einem Atemfluss, einem Atemvolumen, einer Atemfrequenz, einer Einatmungs- und Ausatmungsdauer, einer Atemkontur, einer Leckage oder einem Therapiedruck in Zusammenhang stehen, zu erfassen. Optional kann die Sensoreinheit 23 zusätzliche Messungen von Bestandteilen oder Temperatur des Atemgases oder des Blutes vornehmen. Die Sensoreinheit 23 übermittelt die erfassten Messwerte an das Aufbereitungseinheit 24.

Das Aufbereitungseinheit 25 ist beispielsweise als kombinierte Aufbereitungs- und Berechnungseinheit ausgebildet und kann die erfassten Messwerte aufbereiten. Beispielsweise kann die Aufbereitungseinheit 25 eine Glättung, eine Artefaktbereinigung oder ein Downsampling der Messwerte durchführen. Weiter ist die Aufbereitungseinheit 25 dazu ausgebildet, aus den durch die Sensoreinheit 23 erfassten aufbereiteten Messwerten Signale und/oder Kenngrößen, wie beispielsweise einen Mittelwert, einen Median, ein Perzentil, eine Ableitung, eine Häufigkeitsverteilung, eine Dauer oder einen Anteil eines Über- oder Unterschreitens von Schwellenwerten zu berechnen.

Das Erkennungseinheit 26 ist eingerichtet, Ereignisse/Zustände wie beispielsweise Alarme, Atemaussetzer, Artefakte, Hustenstöße, Sauerstoff(ent)sättigungen, Asynchronien zwischen Gerät und Anwender und/oder spontane Atemzüge zu erkennen.

Die Speichereinheit 24 speichert unter anderem die durch die Sensoreinheit 23 erfassten Werte/Parameter und/oder die durch die Aufbereitungseinheit 25 aufbereiteten Werte, Daten und/oder Informationen, beziehungsweise speichert diese zumindest zwischen. Auch die durch die Erkennungseinheit 26 gewonnenen Informationen, Daten und Werte können und/oder werden in der Speichereinheit zumindest zwischengespeichert. Eine Zwischenspeicherung bedeutet zum Beispiel, dass die Werte, Daten und/oder Informationen bis zu einer Übertragung gespeichert und danach zum Beispiel gelöscht oder zum Überschreiben freigegeben werden.

Die Überwachungseinheit 27 erfasst zum Beispiel technische Probleme des Beatmungsgerätes 2. Technische Probleme können beispielsweise ein niedriger Akkustand, Fehler in der Elektronik, ein defekter Akku, ein defektes Bauteil, ein Stromausfall, ein nicht korrekt funktionierendes Zubehörteil, ein unplausibler Messwert oder ein Verlassen eines erlaubten Temperaturbereichs sein. Die Überwachungseinheit 27 kann bei einem erkannten technischen Problem einen Alarm auf dem Beatmungsgerät 2 über eine Schnittstelle anzeigen oder übermitteln.

Die EIT-Messeinrichtung 3 ist beispielsweise über eine Schnittstelle (nicht dargestellt) mit dem Beatmungsgerät 2 verbunden. In manchen Ausführungsformen kann die EIT-Messeinrichtung 3 auch zumindest teilweise in das Beatmungsgerät 2 integriert sein. Die EIT-Messeinrichtung 3 verfügt zumindest über eine Sensorvorrichtung 31 sowie eine Berechnungs- und Auswerteeinheit 32.

Die Sensorvorrichtung 31 umfasst mehrere Oberflächenelektroden, welche auf der Haut des Lebewesens 4 angeordnet werden. In der Regel umfasst die Sensorvorrichtung mindestens 4 Oberflächenelektroden, wobei häufig zwischen 15 und 65 Oberflächenelektroden eingesetzt werden. Eine Anzahl von mehr als 64 Oberflächenelektroden ist aber auch denkbar. Durch zwei der Oberflächenelektroden, beispielsweise zwei benachbarte Oberflächenelektroden, fließt ein Wechselstrom mit einer niedrigen Amplitude sowie einer niedrigen Stromstärke (in der Regel im Bereich von einstelligen Milliampere-Werten). Dieser Wechselstrom wird von den anderen Oberflächenelektroden, beispielsweise in Form von Impedanzwerten, gemessen. Beispielsweise ist die EIT-Messeinrichtung 3 so eingerichtet, dass die Oberflächenelektroden, zwischen denen der Wechselstrom fließt, nach der Messung wechseln, beispielsweise zu den benachbarten zwei Oberflächenelektroden, wobei sich dieser Vorgang beispielsweise so lange wiederholt bis wieder zwischen den ersten beiden Oberflächenelektroden der Wechselstrom fließt. Beispielsweise ist damit ein Messzyklus abgeschlossen. In manchen Ausführungsformen der EIT-Messeinrichtung 3 fließt der Strom immer zwischen den zwei gleichen Oberflächenelektroden und wird immer von den gleichen anderen Oberflächenelektroden gemessen.

Die EIT-Messeinrichtung 3 ist weiter so ausgebildet, dass, beispielsweise über die Berechnungs- und Auswerteeinheit 32, die von der Sensorvorrichtung 31 gemessenen Impedanzwerte individuellen Pixeln zugeordnet werden können. Weiter ist die Berechnungs- und Auswerteeinheit 32 dazu ausgebildet aus den Impedanzwerten verschiedene Kennzahlen zu berechnen sowie basierend auf den Kennzahlen eine Auswertung durzuführen und beispielsweise Empfehlungen bezüglich der Beatmungseinstellungen, wie zum Beispiel den inspiratorischen Druck $P_{insp}$ und/oder den positiven end-exspiratorischen Druck PEEP und/oder dem exspiratorischen Tidalvolumen VT, zu generieren.

Das System 1 ist beispielsweise so eingerichtet, dass die EIT-Messeinrichtung 3 und/oder das Beatmungsgerät 2 zu zumindest zwei Zeitpunkten eines durch das Beatmungsgerät 2 durchgeführten Beatmungsmanöver Impendanzwerte misst, diese Impedanzwerte auswertet beziehungsweise mit den Impedanzwerten Kennzahlen zu berechnen und daraufhin unter Berücksichtigung der Einstellungen des Beatmungsmanövers Empfehlungen zu den Beatmungseinstellungen zu erstellen und gegebenenfalls auszugeben, beispielsweise über die Anzeigevorrichtung des Beatmungsgerätes 2 oder eine Schnittstelle, über welche zum Beispiel ein Telemonitoringsystem angeschlossen ist.

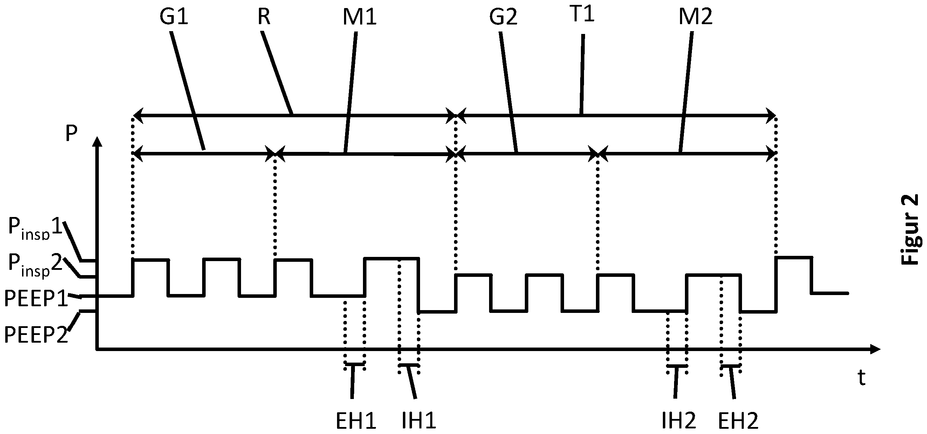

Anhand der Figuren 2 bis 4 soll das Beatmungsmanöver sowie die Auswertung der Impedanzmessungen beispielhaft erläutert werden.

In Figur 2 ist ein beispielhafter Ablauf des Beatmungsmanövers zu sehen, welche am Beatmungsgerät 2 eingestellt werden kann. Die x-Achse stellt dabei den Druckwert P dar und die y-Achse die Zeit t. Im Wesentlichen setzt sich das Beatmungsmanöver aus zwei Phasen, der Referenzphase R und einer Testphase T1, zusammen. Die Testphase T1 unterscheidet sich von der Referenzphase R zumindest darin, dass eine Beatmungseinstellung verschieden ist. In manchen Ausführungsformen können auch noch weitere Testphasen Tm eingestellt werden, wobei sich jede weitere Testphase ebenfalls in zumindest einer Beatmungseinstellung von allen anderen Referenzphasen R und Testphasen T1, Tm unterscheidet. Hierbei bezeichnet "m" die jeweilige Nummer der Testphase, die zweite Testphase würde entsprechend mit T2 bezeichnet und so weiter. Beispielsweise unterscheiden sich die Referenzphase R und die Testphase T1 in dem positiven end-exspiratorischen Druck (PEEP) und/oder dem inspiratorischen Druck $P_{insp}$. Beispielsweise kann am Beatmungsgerät 2 eingestellt werden, welche Beatmungseinstellung und in welcher Höhe sich die Beatmungseinstellung zwischen Referenzphase R und Testphase T1 unterscheidet. Für das in Figur 2 beispielhaft gezeigte Manöver ist beispielsweise ein reduzierter PEEP für die Testphase T1 vorgesehen (mit PEEP2 gekennzeichnet), wobei die Druckdifferenz $\Delta P$ zwischen dem PEEP und dem inspiratorischen Druck $P_{insp}$ gleichbleibt, was zugleich zur Folge hat, dass der inspiratorische Druck $P_{insp}$ in der Testphase T1 (mit $P_{insp}2$ gekennzeichnet) um den gleichen Wert reduziert wird wie der PEEP. Neben einer Reduzierung des PEEP kann auch ein Erhöhen des PEEP eingestellt werden. Auch ein Reduzieren/Erhöhen der Druckdifferenz $\Delta P$ (Differenz von $P_{insp}$ und PEEP) oder ein Reduzieren/Erhöhen mittels eines Faktors des eingestellten Tidalvolumen kann für das Beatmungsmanöver eigestellt werden, wobei dabei beispielsweise der PEEP konstant gehalten wird. In manchen Ausführungsformen kann auch eine Erhöhung/Reduzierung des PEEP eingestellt werden, wobei der inspiratorische Druck $P_{insp}$ konstant bleibt, also $\Delta P$ entsprechend erhöht/reduziert wird. Während der Referenzphase R wird gemäß dem Manöver in Figur 2 das Lebewesen 4 mit einem positiven end-exspiratorischen Druck PEEP1 und einem inspiratorischen Druck $P_{insp}1$ beatmet beziehungsweise bei der Atmung unterstützt. Während der Testphase T1 ändert sich der PEEP bei im Vergleich mit der Referenzphase gleichbleibender Druckdifferenz ΔP. Das Lebewesen 4 wird als mit einem positiven end-exspiratorischen Druck PEEP2 und einem inspiratorischen Druck $P_{insp}2$ beatmet beziehungsweise bei der Atmung unterstützt.

Jede der Phasen des Beatmungsmanövers umfasst mehrere Atemzüge, bestehend aus Inspiration und Exspiration. Beispielsweise können jeweils für die Referenzphase R und jede der Testphasen T1, Tm eine Anzahl an Atemzügen von 5 bis 120, bevorzugt 5 bis 60, eingestellt werden. Die Referenzphase R sowie die Testphase T1 umfassen zudem zumindest zwei Unterphasen - jeweils eine Gewöhnungsphase G1, G2 und eine Messphase M1, M2. Die Gewöhnungsphase G1, G2 dient dazu, dass sich das Lebewesen 4 an die Beatmungseinstellungen gewöhnen kann. Die Messphasen M1, M2 schließen sich an die jeweilige Gewöhnungsphase G1, G2 an und umfassen in der Regel zwei Atemzüge, wobei die Messphase M1, M2 auch mehr Atemzüge umfassen kann. Je nach Definition - also was als Beginn eines Atemzuges angesehen wird - kann die Messphase auch nur einen Atemzug umfassen. Während der Messphase M1, M2 werden zu zumindest zwei Zeitpunkten durch die EIT-Messeinrichtung 3 Impedanzwerte aufgenommen, wobei ein Zeitpunkt auch mit der Zeitspanne gleichgesetzt werden kann, welche für einen Messzyklus der EIT-Messeinrichtung 3 benötigt wird.

Gemäß dem in Figur 2 dargestellten beispielhaften Ablauf des Beatmungsmanövers wird zum Ende der Exspiration des vorletzten Atemzuges der jeweiligen Referenzphase R bzw. Testphase T1 ein end-exspiratorisches Hold-Manöver EH1, EH2 durchgeführt. Bei dem end-exspiratorischen Hold-Manöver EH1, EH2 wird zum Ende der Exspiration das Druckniveau des PEEP gehalten. Während diesem Hold-Manöver EH1, EH2 liegt der jeweils erste Zeitpunkt, zu dem die EIT-Messeinrichtung 3 die Impedanzwerte aufnimmt bzw. misst.

Nach dem vorletzten Atemzug folgt zum Ende der Inspiration des letzten Atemzuges der jeweiligen Messphase M1, M2 ein inspiratorisches Hold-Manöver IH1, IH2, bei welchem das Druckniveau des inspiratorischen Druckes $P_{insp}1$, $P_{insp}2$ gehalten wird. Analog zu den exspiratorischen Hold-Manövern EH1, EH2 liegt ein Zeitpunkt, zu dem die EIT-Messeinrichtung 3 Impedanzwerte misst bzw. aufnimmt während der inspiratorischen Hold-Manöver IH1, IH2.

Die Länge der Hold-Manöver EH1, EH2, IH1, IH2 ist dabei beispielsweise über das Beatmungsgerät 2 und/oder die EIT-Messeinrichtung 3 einstellbar. Die Längen können Beispielsweise in einem Bereich von 0,2 Sekunden bis 8 Sekunden eingestellt werden, wobei die jeweiligen inspiratorischen Hold-Manöver IH1, IH2 gleichlang sind und die jeweiligen exspiratorischen Hold-Manöver EH1, EH2 gleich lang sind. Die inspiratorischen Hold-Manöver müssen dabei allerdings nicht die gleiche Länge aufweisen wie die exspiratorischen Hold-Manöver.

Auch die Veränderung des PEEP, $P_{insp}$ und/oder ΔP der Testphase T1 gegenüber der Referenzphase R können beispielsweise über das Beatmungsgerät 2 und/oder die EIT-Messeinrichtung 3 eingegeben werden. Beispielsweise kann eine absolute Veränderung in mbar eingegeben werden und/oder auch eine relative Veränderung als Faktor eingestellt werden.

In manchen Ausführungsformen des Systems 1 ist es auch möglich die Einstellungen über ein über eine Schnittstelle mit dem System 1 verbundenes Gerät, beispielsweise eine Telemonitoring-Einheit, vorzunehmen.

Die gemessenen Impedanzwerte werden, beispielsweise durch die Berechnungs- und Auswerteeinheit 32, individuellen Pixeln n zuzuordnen, wobei n hier eine Bezeichnung, beispielsweise fortlaufende Nummerierung, der Pixel darstellt. Bei einer Gesamtzahl von 1024 Pixeln ergibt sich so beispielsweise eine Zählung für n von 1 bis 1024. Die Berechnungs- und Auswerteeinheit 32 ist weiter dazu ausgebildet, je nach Phase (Referenz- und Testphase) sowie je nach Messzeitpunkt (EH1, IH1, EH2, IH2) die Impedanzwerte entsprechend gleichbleibend dem Pixel zuzuordnen. Demnach sind beispielsweise nach Abschluss des Beatmungsmanövers aus Figur 2 jedem Pixel zumindest vier Impedanzwerte I (jeweils einer aus EH1, IH1, EH2, IH2) zugeordnet.

Die Berechnungs- und Auswerteeinheit 32 ist weiter dazu ausgebildet, aus den Impedanzwerten der jeweiligen Referenz- bzw. Testphase für jedes Pixel je eine Kennzahl $C_{R,n}$ für die Referenzphase R und eine Kennzahl $C_{T1,n}$ für die Testphase T1 zu berechnen. Dazu wird von der Berechnungs- und Auswerteeinheit 32 zunächst für jede Phase R, T1 die Differenz $\Delta I_n$ zwischen $I_{Max,n}$ und $I_{Min,n}$ gebildet, wobei $I_{Max,n}$ der jeweilige Impedanzwert des Pixels während der Messung während des exspiratorischen Hold-Manövers EH1 bzw. EH2 und $I_{Min,n}$ der jeweilige Impedanzwert des Pixels während der Messung während des inspiratorischen Hold-Manövers IH1 bzw. IH2 darstellt:

$$\Delta I_n = I_{Max,n} - I_{Min,n}$$

In manchen Ausführungsformen werden individuelle Pixel nur dann weiter mit in die Berechnung und Auswertung einbezogen, wenn $\Delta I_n$ größer als Null ist. Weiter wird die Differenz $\Delta I_{global}$ zwischen $I_{Max,global}$ und $I_{Min,global}$ gebildet, wobei $I_{Max,global}$ die Summe der Impedanzwerte aller Pixel während der Messung während des exspiratorischen Hold-Manövers EH1 bzw. EH2 und $I_{Min,global}$ die Summe der Impedanzwerte aller Pixel während der Messung während des inspiratorischen Hold-Manövers IH1 bzw. IH2 darstellt:

$$\Delta I_{global} = I_{Max,global} - I_{Min,global} = \sum I_{Max,n} - \sum I_{Min,n}$$

Ist der Wert $\Delta I_n$ für ein Pixel gleich oder unter Null, wird dieses Pixel auch nicht mit in die Berechnung von $\Delta I_{global}$ einbezogen. Jedes Pixel n erhält also zwei Werte $\Delta I_n$ - einen für die Referenzphase R und einen für die Testphase T1. Ebenso wird entsprechend für die Referenzphase R ein Wert $\Delta I_{global}$ berechnet als auch für die Testphase T1. Durch die Berechnungs- und Auswerteeinheit 32 erfolgt folgend die Berechnung der Kennzahlen $C_{R,n}$ und $C_{T1,n}$ für die jeweilige Phase R bzw. T1 gemäß

$$C_{R,n} = \frac{\Delta I_n}{\Delta I_{global}} \cdot C_{R,Stat}$$

$$C_{T1,n} = \frac{\Delta I_n}{\Delta I_{global}} \cdot C_{T1,Stat}$$

wobei die Faktoren $C_{R,Stat}$ und $C_{T1,Stat}$ aus dem beispielsweise durch die EIT-Messeinrichtung 3 oder das Beatmungsgerät 2 ermittelten Tidalvolumen VT und/oder einem Exspirationsvolumen sowie der Druckdifferenz zwischen dem inspiratorischen Hold-Manöver IH1 bzw. IH2 sowie dem exspiratorischen Hold-Manöver EH1 bzw. EH2 berechnet werden und statistische Compliance-Werte wiedergeben. Bezogen auf Figur 2 wird also die Differenz zwischen $P_{insp}1$ und PEEP1 für $C_{R,Stat}$ und die Differenz zwischen $P_{insp}2$ und PEEP2 für $C_{T1,Stat}$ verwendet.

Nach Berechnung werden die Kennzahlen $C_{R,n}$ und $C_{T1,n}$ der individuellen Pixel miteinander verglichen und einer von zwei Zahlengruppen zugeordnet. Die beiden Zahlengruppen sind beispielsweise mit WIN und LOSS bezeichnet. Die Pixel, für die gilt, dass $C_{T1,n}$ größer ist als $C_{R,n}$ werden durch die Berechnungs- und Auswerteeinheit 32 der Zahlengruppe WIN zugeordnet, währen die Pixel, für die gilt, dass $C_{T1,n}$ kleiner ist als $C_{R,n}$ der Gruppe LOSS zugeordnet werden. Ein größerer Wert für $C_{T1,n}$ als $C_{R,n}$ kann beispielsweise als Compliance-Gewinn des einzelnen Pixels in der Testphase T1 gegenüber der Referenzphase R angesehen werden.

Für einen weiteren Schritt ist die Berechnungs- und Auswerteeinheit 32 dazu ausgebildet, mit den Kennzahlen $C_{R,n}$ und $C_{T1,n}$ der Pixel der Zahlengruppe WIN eine Kennzahl $C_{WIN,global}$ und mit mit den Kennzahlen $C_{R,n}$ und $C_{T1,n}$ der Pixel der Zahlengruppe LOSS eine Kennzahl $C_{LOSS,global}$ zu berechnen.

Die Kennzahlen $C_{WIN,global}$ und $C_{LOSS,global}$ berechnen sich jeweils durch die Summe aller Kennzahlen für den Compliance-Gewinn $C_{WIN,n}$ der Pixel aus der Zahlengruppe WIN multipliziert mit 100 beziehungsweise durch die Summe aller Kennzahlen für den Complinance-Verlust $C_{LOSS,n}$ der Pixel aus der Zahlengruppe LOSS multipliziert mit 100 und geben einen auf das Lebewesen bezogenen globalen Compliance-Gewinn (WIN) beziehungsweise Compliance-Verlust (LOSS) wieder:

$$C_{WIN,global} = \sum C_{WIN,n} \cdot 100$$

$$C_{LOSS,global} = \sum C_{LOSS,n} \cdot 100$$

Die Kennzahlen $C_{WIN,global}$ und $C_{LOSS,global}$ geben letztlich globale Compliance-Gewinne bzw. Verluste in Prozent, beispielsweise bezogen auf die statistische Compliance $C_{R,Stat}$, an. Dabei errechnet die Berechnungs- und Auswerteeinheit 32 den Compliance-Gewinn $C_{WIN,n}$ bzw. den Compliance-Verlust $C_{LOSS,n}$ jedes einzelnen Pixels anhand

$$C_{WIN,n} = \frac{C_{T1,n} - C_{R,n}}{C_{R,Stat}}$$

$$C_{LOSS,n} = \frac{C_{T1,n} - C_{R,n}}{C_{R,Stat}}$$

unter Verwendung von $C_{T1,n}$ und $C_{R,n}$ der Pixel der jeweiligen Zahlengruppe WIN und LOSS.

Insbesondere die Kennzahlen des globalen Compliance-Verlustes $C_{LOSS,global}$ und des globalen Compliance-Gewinns $C_{WIN,global}$ können durch die Berechnungs- und Auswerteeinheit 32 dazu genutzt werden, die Beatmung und/oder die Beatmungseinstellungen des Beatmungsgerätes 2 zu bewerten. Beispielsweise überprüft die Berechnungs- und Auswerteeinheit 32 dazu, ob die Kennzahlen $C_{WIN,global}$ und/oder $C_{LOSS,global}$ definierbare Schwellenwerte überschreiten. Die Schwellenwerte können beispielsweise im System programmierbar sein und einen Bereich von 1% bis 99%, beispielsweise in einem Bereich von 10% bis 50%. In manchen Ausführungsformen können die Schwellenwerte auch unabhängig voneinander eingestellt werden. Die Bewertung durch die Berechnungs- und Auswerteeinheit 32 erfolgt daraufhin, ob und welche der Kennzahlen $C_{WIN,global}$ und $C_{LOSS,global}$ den jeweiligen Schwellenwert erreichen beziehungsweise überschreiten. Insgesamt ergeben sich dabei vier mögliche Ergebnisse:

- Sowohl $C_{WIN,global}$ als auch $C_{LOSS,global}$ liegen unter dem Schwellenwert
- $C_{WIN,global}$ liegt über dem Schwellenwert und $C_{LOSS,global}$ liegt unter dem Schwellenwert
- $C_{WIN,global}$ liegt unter dem Schwellenwert und $C_{LOSS,global}$ liegt über dem Schwellenwert
- $C_{WIN,global}$ und $C_{LOSS,global}$ liegen über dem Schwellenwert.

Das System 1 ist allgemein dazu ausgebildet, eine alphanumerische und/oder graphische Auswertung der Kennzahlen auszugeben. Beispielsweise kann die Auswertung beziehungsweise die Ausgabe der Kennzahlen in Form eines Balkendiagramms ausgegeben werden, wobei die Balkengröße der Größe der jeweiligen Kennzahl, beispielsweise auch im Verhältnis zur statistischen Compliance $C_{R,Stat}$ entspricht. Auch eine Darstellung als Kuchendiagramm ist beispielsweise denkbar.

Unter Verwendung der globalen Compliance-Verluste und -Gewinne und der Einstellungen des Beatmungsmanövers können durch das System 1 bezüglich der Beatmungseinstellungen Empfehlungen ausgegeben werden. Um eine solche Aussage zu ermöglichen sollte die Referenzphase R des Beatmungsmanövers den Beatmungseinstellungen der bisherigen Beatmung entsprechen. Wie bereits beschrieben unterscheidet sich die Testphase T1 in zumindest einer Beatmungseinstellung von der Referenzphase R. Insbesondere auf Basis dieser unterscheidenden Beatmungseinstellung ist das System 1 ausgebildet, die Empfehlungen bezüglich der Beatmungseinstellungen zu generieren und/oder auszugeben.

Die globalen Compliance-Verluste $C_{LOSS,global}$ und Compliance-Gewinne $C_{WIN,global}$ können zudem auch genutzt werden um einen Befund zu erstellen. Ebenso wie die Empfehlungen, sollte die Referenzphase R des Beatmungsmanövers den Beatmungseinstellungen der bisherigen Beatmung entsprechen, um eine entsprechende Erstellung eines Befundes zu ermöglichen. In manchen Ausführungsformen wird alternativ auch zunächst der Befund erstellt und auf Basis dieses Befundes werden die Empfehlungen generiert beziehungsweise ausgegeben.

Die Befunde werden beispielsweise über eine Anzeige alphanumerisch und/oder graphisch ausgegeben, beispielsweise gemeinsam mit der Empfehlung bezüglich der Beatmungseinstellungen.

Entsprechend der beschriebenen beispielhaft beschriebenen Einstellmöglichkeiten für das Beatmungsmanöver (Erhöhen/Reduzieren von PEEP, $P_{insp}$, VT, $\Delta P$ bei gleichbleibendem $P_{insp}$) und der möglichen Ergebnisse der Auswertung der Kennzahlen $C_{WIN,global}$ und $C_{LOSS,global}$ ergeben sich beispielsweise folgende Empfehlungsmöglichkeiten. Die Empfehlungen werden beispielsweise alphanumerisch ausgegeben.

Ist der PEEP der Testphase T1 gegenüber dem PEEP der Referenzphase R erhöht, gilt

a. wenn $C_{WIN,global}$ und $C_{LOSS,global}$ unter dem eingestellten Schwellenwert liegen, wird die Empfehlung ausgegeben, die Beatmungseinstellungen nicht zu verändern, zudem kann ein Befund erstellt werden, dass keine signifikanten Änderungen festgestellt wurden,

b. wenn $C_{WIN,global}$ über dem eingestellten Schwellenwert liegt und $C_{LOSS,global}$ unter dem eingestellten Schwellenwert liegt, wird die Empfehlung ausgegeben, den PEEP zu erhöhen, zudem kann ein Befund erstellt werden, dass es zu einer Rekrutierung bei Erhöhung des PEEP kommt,

c. wenn $C_{WIN,global}$ unter dem eingestellten Schwellenwert liegt und $C_{LOSS,global}$ über dem eingestellten Schwellenwert liegt, wird die Empfehlung ausgegeben, die den PEEP nicht zu verändern und eine Reduzierung von $P_{insp}$ und/oder VT zu erwägen, wobei zunächst das Beatmungsmanöver inklusive Messungen mit reduziertem $P_{insp}$ in der Testphase T1 durchgeführt werden sollte, zudem kann ein Befund erstellt werden, dass es durch die Erhöhung des PEEP zu einer Überdehnung kommt,

d. wenn $C_{WIN,global}$ und $C_{LOSS,global}$ über dem eingestellten Schwellenwert liegen, wird die Empfehlung ausgegeben, den PEEP zu erhöhen und eine Reduzierung von $P_{insp}$ und/oder VT zu erwägen, wobei zunächst das Beatmungsmanöver inklusive Messungen mit reduziertem $P_{insp}$ in der Testphase T1 durchgeführt werden sollte, zudem kann ein Befund erstellt werden, dass es durch die Erhöhung des PEEP sowohl zu einer Rekrutierung als auch zu einer Überdehnung kommt.

Ist der PEEP der Testphase T1 gegenüber dem PEEP der Referenzphase R reduziert, gilt

a. wenn $C_{WIN,global}$ und $C_{LOSS,global}$ unter dem eingestellten Schwellenwert liegen, wird die Empfehlung ausgegeben, die Beatmungseinstellungen nicht zu verändern und/oder den PEEP zu reduzieren, zudem kann ein Befund erstellt werden, dass keine signifikanten Änderungen festgestellt wurden,

b. wenn $C_{WIN,global}$ über dem eingestellten Schwellenwert liegt und $C_{LOSS,global}$ unter dem eingestellten Schwellenwert liegt, wird die Empfehlung ausgegeben, den PEEP zu reduzieren, zudem kann ein Befund erstellt werden, dass eine nachlassende Überdehnung zu beobachten ist,

c. wenn $C_{WIN,global}$ unter dem eingestellten Schwellenwert liegt und $C_{LOSS,global}$ über dem eingestellten Schwellenwert liegt, wird die Empfehlung ausgegeben, die Einstellung des PEEP nicht zu verändern und/oder zu erhöhen und ein Rekrutierungsmanöver zu erwägen, zudem kann ein Befund erstellt werden, dass eine Derekrutierung zu beobachten ist,

d. wenn $C_{WIN,global}$ und $C_{LOSS,global}$ über dem eingestellten Schwellenwert liegen, wird die Empfehlung ausgegeben, die Einstellung des PEEP nicht zu verändern und ein Rekrutierungsmanöver zu erwägen sowie, soweit möglich, $P_{insp}$ und/oder VT zu reduzieren, zudem kann ein Befund erstellt werden, dass sowohl eine nachlassende Überdehnung als auch eine Derektutierung zu beobachten ist.

Ist der inspiratorische Druck $P_{insp}$ in der Testphase T1 gegenüber $P_{insp}$ der Referenzphase R erhöht, gilt

a. wenn $C_{WIN,global}$ und $C_{LOSS,global}$ unter dem eingestellten Schwellenwert liegen, wird die Empfehlung ausgegeben, die Beatmungseinstellungen nicht zu verändern, zudem kann ein Befund erstellt werden, dass keine signifikanten Änderungen zu beobachten sind,

b. wenn $C_{WIN,global}$ über dem eingestellten Schwellenwert liegt und $C_{LOSS,global}$ unter dem eingestellten Schwellenwert liegt, wird die Empfehlung ausgegeben, den PEEP zu erhöhen, zudem kann ein Befund erstellt werden, dass der Verdacht einer tidalen Rekrutierung zu beobachten ist,

c. wenn $C_{WIN,global}$ unter dem eingestellten Schwellenwert liegt und $C_{LOSS,global}$ über dem eingestellten Schwellenwert liegt, wird die Empfehlung ausgegeben, die Einstellung des PEEP und/oder des $P_{insp}$ und/oder von VT zu reduzieren, zudem kann ein Befund erstellt werden, dass eine Überdehnung zu beobachten ist,

d. wenn $C_{WIN,global}$ und $C_{LOSS,global}$ über dem eingestellten Schwellenwert liegen, wird die Empfehlung ausgegeben, den PEEP zu erhöhen und/oder, soweit möglich, den $P_{insp}$ und/oder das Tidalvolumen zu reduzieren, zudem kann ein Befund erstellt werden, dass eine Überdehnung und der Verdacht einer tidalen Rekrutierung zu beobachten ist.

Ist der inspiratorische Druck $P_{insp}$ in der Testphase T1 gegenüber $P_{insp}$ der Referenzphase R reduziert, gilt

a. wenn $C_{WIN,global}$ und $C_{LOSS,global}$ unter dem eingestellten Schwellenwert liegen, wird die Empfehlung ausgegeben, die Beatmungseinstellungen nicht zu verändern, zudem kann ein Befund erstellt werden, dass keine signifikanten Änderungen zu beobachten sind,

b. wenn $C_{WIN,global}$ über dem eingestellten Schwellenwert liegt und $C_{LOSS,global}$ unter dem eingestellten Schwellenwert liegt, wird die Empfehlung ausgegeben, den PEEP und/oder $P_{insp}$ und/oder VT zu reduzieren, zudem kann ein Befund erstellt werden, dass eine nachlassende Überdehnung zu beobachten ist,

c. wenn $C_{WIN,global}$ unter dem eingestellten Schwellenwert liegt und $C_{LOSS,global}$ über dem eingestellten Schwellenwert liegt, wird die Empfehlung ausgegeben, den PEEP zu erhöhen, zudem kann ein Befund erstellt werden, dass der Verdacht auf eine tidale Rekrutierung zu beobachten ist,

d. wenn $C_{WIN,global}$ und $C_{LOSS,global}$ über dem eingestellten Schwellenwert liegen, wird die Empfehlung ausgegeben, den PEEP zu erhöhen und/oder, soweit möglich, den $P_{insp}$ und/oder das Tidalvolumen zu reduzieren, zudem kann ein Befund erstellt werden, dass eine nachlassende Überdehnung und ein Verdacht auf eine tidale Rekrutierung zu beobachten ist.

In manchen Ausführungsformen ist das System 1 dazu eingerichtet, die Empfehlungen automatisch umzusetzen und die Beatmungseinstellungen entsprechend der Auswertung und Empfehlung anzupassen. Auch kann das System 1 beispielsweise dazu eingerichtet sein, beispielsweise ein empfohlenes Rekrutierungsmanöver und/oder weitere Beatmungsmanöver inklusive Messungen durchzuführen.

Beispielsweise ist es auch denkbar, dass das System 1 mehrere Beatmungsmanöver und Messungen mit den gleichen Einstellungen durchführt und anhand eines Durschnitts oder Mittelwerts der Ergebnisse eine entsprechende Empfehlung generieren und ausgeben.

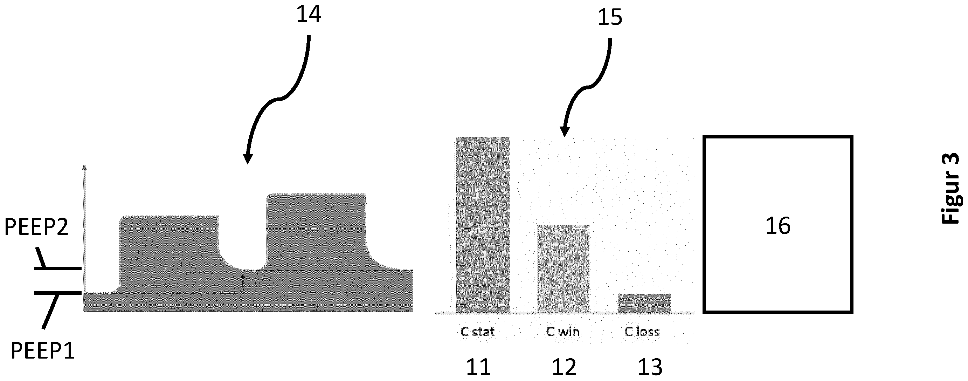

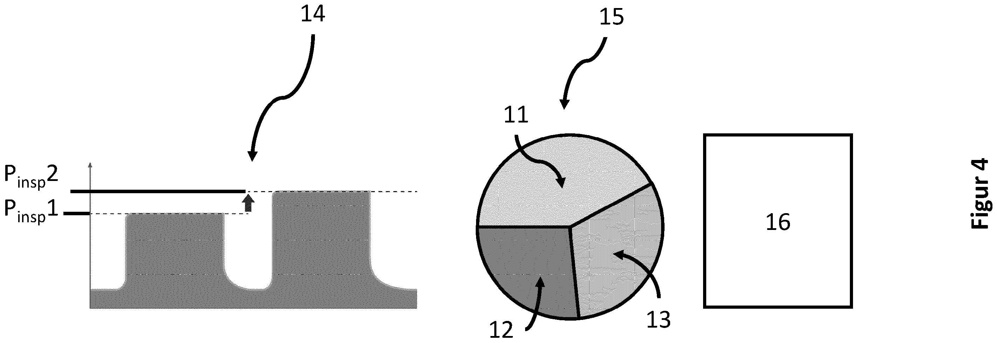

Die Figuren 3 und 4 zeigen eine beispielhafte Ausführungsform der alphanumerischen und visuellen Ausgabe der Auswertung und Empfehlungen des Systems 1. In der Ausführungsform der Figur 3 ist beispielhaft eine visuelle Darstellung 14 der gewählten Manövereinstellung (hier ein erhöhter PEEP in der Testphase T1) neben der graphischen Ausgabe 15 der Auswertung der Kennzahlen $C_{WIN,global}$ (12), $C_{LOSS,global}$ (13) und $C_{R,Stat}$ (11) und der alphanumerischen Ausgabe der Empfehlungen 16 zu sehen. In dem Feld der Empfehlungen 16 kann ergänzend dazu auch ein Befund angezeigt werden, welcher beispielsweise auf Basis der Kennzahlen $C_{WIN,global}$ (12) und $C_{LOSS,global}$ (13) und beispielsweise automatisch durch das System (1), erstellt wird. In dem gezeigten Fall wird die Auswertung der Kennzahlen als Balkendiagramm graphisch dargestellt. Beispielhaft liegt die Kennzahl $C_{WIN,global}$ über dem eingestellten Schwellenwert und die Kennzahl $C_{LOSS,global}$ unter dem eingestellten Schwellenwert. In der alphanumerischen Ausgabe der Empfehlungen 16 sollte hier entsprechend empfohlen werden den PEEP der Beatmungseinstellungen zu erhöhen um eine effizientere und/oder bessere Beatmung zu erreichen.

Figur 4 zeigt beispielhaft eine weitere möglich alphanumerische und graphische Darstellung des Beatmungsmanövers 14, der Auswertung der Kennzahlen 15 sowie der Empfehlungen 16. Hier gezeigt wird beispielsweise, dass sowohl $C_{WIN,global}$ (12) als auch $C_{LOSS,global}$ (13) über dem eingestellten Schwellenwert liegen, wobei die Beatmungsmanövereinstellung einen erhöhten inspiratorischen Druck $P_{insp}$ für die Testphase T1 vorgesehen hat. Das System gibt daher beispielsweise im Feld für die Empfehlung 16 einen alphanumerischen Text aus, der besagt, dass der PEEP zu erhöhen und, soweit möglich, der $P_{insp}$ und/oder das Tidalvolumen zu reduzieren wären.

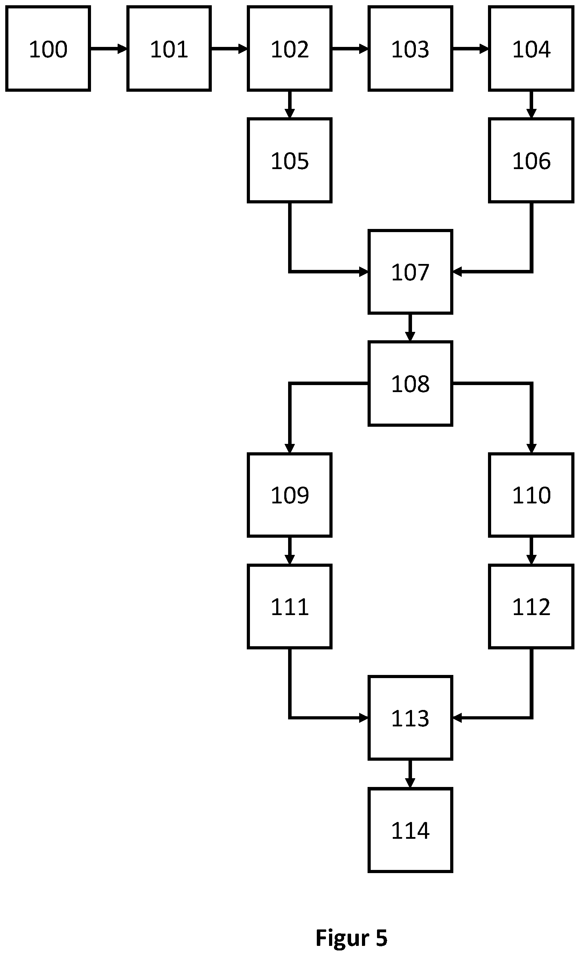

Figur 5 zeigt beispielhaft und schematisch einen Ablauf der Schritte, welche vom System durchgeführt werden um basierend auf den von der EIT-Messeinrichtung gemessenen Impedanzwerten sowie dem Beatmungsmanöver (vgl. Figur 2) Empfehlungen zu den Beatmungseinstellungen zu generieren.

Der erste Schritt stellt dabei das Einstellen 100 des Beatmungsmanövers dar, wobei unter anderem die Beatmungseinstellung gewählt wird, in welcher sich die Testphase T1 von der Referenzphase R unterscheidet sowie die Höhe des Unterschieds. Auch können während des Einstellens 100 die Länge der inspiratorischen und exspiratorischen Hold-Manöver EH1, EH2, IH1, IH2 sowie die Dauer (beispielsweise in Form von Anzahl der Atemzüge) der jeweiligen Referenzphase R und Testphase T1 gewählt werden. Auch kann während des Einstellens 100 der jeweilige Schwellenwert für die Kennzahlen $C_{WIN,global}$ und $C_{LOSS,global}$ festgelegt werden.

Dem Einstellen 100 folgt der Schritt des ersten Gewöhnens 101 des Lebewesens an die Beatmungseinstellungen der Referenzphase R. Anschließend an das Gewöhnen 101 folgt das Messen 102 in der Referenzphase R. Dabei werden während der letzten Atemzüge der Referenzphase R Impedanzwerte aufgenommen.

Dem Schritt des Messens 102 schließt sich der Schritt des Gewöhnens 103 an, welcher dem Gewöhnen 103 des Lebewesens an die Beatmungseinstellungen der Testphase T1 dient. Während der Testphase T1 wird die eingestellte Veränderung der Beatmungseinstellungen aus dem Schritt des Einstellens 100 angewendet. Auf den Schritt des Gewöhnens 103 folgt der Schritt des Messens 104 der Impedanzwerte während der Testphase T1.

Den Schritten des Messens 102, 104 schließen sich jeweils die Schritte des Berechnens 105, 106 an. Das Berechnen 105 umfasst dabei die Berechnung der Kennzahl $C_{R,n}$ für jedes Pixel aus den während des Messens 102 gemessenen Impedanzwerten der Referezphase R1. Das Berechnen 106 umfasst dabei die Berechnung der Kennzahl $C_{T1,n}$ für jedes Pixel aus den während des Messens 104 gemessenen Impedanzwerten der Testphase T1.

Das folgende Vergleichen 107 umfasst den Vergleich der Kennzahlen $C_{R,n}$ und $C_{T1,n}$. Basierend auf den Ergebnissen des Vergleichens 107 erfolgt das Einordnen 108 der Pixel und der damit zusammenhängenden Kennzahlen $C_{R,n}$ und $C_{T1,n}$ in die Zahlengruppen WIN beziehungsweise LOSS.

Nach dem Einordnen 108 führt das System 1 die Schritte des Berechnens 109, 110 durch, wobei das Berechnen 109 die Berechnung der Kennzahl $C_{WIN,global}$ umfasst und das Berechnen 110 die Berechnung der Kennzahl $C_{LOSS,global}$ umfasst.

Die Kennzahlen $C_{WIN,global}$ und $C_{LOSS,global}$ werden in den folgenden Schritten des Vergleichens 111, 112 mit den jeweils eingestellten Schwellenwerten verglichen, wobei während des Vergleichens 111 die Kennzahl $C_{WIN,global}$ mit dem eingestellten Schwellenwert für $C_{WIN,global}$ verglichen wird und während des Vergleichens 112 Kennzahl $C_{LOSS,global}$ mit dem eingestellten Schwellenwert für $C_{LOSS,global}$ verglichen wird.

Nach dem Vergleichen 111, 112 führt das System 1 den Schritt des Auswertens 113 durch. Während des Auswertens 113 wird beispielsweise eine graphische Darstellung der Kennzahlen $C_{WIN,global}$ und $C_{LOSS,global}$ generiert und ausgewertet, ob beziehungsweise welche der Kennzahlen $C_{WIN,global}$ und $C_{LOSS,global}$ über den eingestellten Schwellenwerten liegen. Der Schritt des Auswertens 113 umfasst beispielsweise auch das Erstellen eines Befundes basierend auf den Kennzahlen $C_{WIN,global}$ (12) und $C_{LOSS,global}$ (13).

Auf Basis der Auswertung aus dem Schritt des Auswertens 113 erfolgt der Schritt des Empfehlens 114, wobei das System 1 entsprechend der Einstellung des Beatmungsmanövers und der Kennzahlen $C_{WIN,global}$ und $C_{LOSS,global}$ beziehungsweise ob und welche der Kennzahlen $C_{WIN,global}$ und $C_{LOSS,global}$ die eingestellten Schwellenwerte überschreiten eine Empfehlung zu den Beatmungseinstellungen generiert und gegebenenfalls als alphanumerische Meldung ausgibt.

Bevorzugt werden die Schritte 101 bis 114 vollautomatisiert vom System 1 durchgeführt, sobald das Einstellen 100 abgeschlossen ist. In manchen Ausführungsformen schließt sich an das Empfehlen 114 beispielsweise noch ein Schritt des Umsetzens an, in dem das System automatisch die generierten Empfehlungen auf die Beatmungseinstellungen anwendet.

Bezugszeichenliste

1        System

| 2 | Beatmungsgerät |
|---|---|
| 3 | EIT-Messeinrichtung |
| 4 | Lebewesen |
| 11 | $C_{R,Stat}$ |
| 12 | $C_{WIN,global}$ |
| 13 | $C_{LOSS,global}$ |
| 14 | Darstellung Beatmungsmanöver |
| 15 | Ausgabe Auswertung |
| 16 | Ausgabe Empfehlung |
| 21 | Atemgasquelle |
| 22 | Steuereinheit |
| 23 | Sensoreinheit |
| 24 | Speichereinheit |
| 25 | Aufbereitungseinheit |
| 26 | Überwachungseinheit |
| 27 | Erkennungseinheit |
| 31 | Sensoreinrichtung |
| 32 | Berechnungs- und Auswerteeinheit |
| 100 | Einstellen |
| 101 | Gewöhnen |
| 102 | Messen |
| 103 | Gewöhnen |
| 104 | Messen |
| 105 | Berechnen |
| 106 | Berechnen |
| 107 | Vergleichen |
| 108 | Einordnen |
| 109 | Berechnen |
| 110 | Berechnen |

EP 4 011 424 B1

| 111 | Vergleichen |
|---|---|
| 112 | Vergleichen |
| 113 | Auswerten |
| 114 | Empfehlen |
| EH1 | exspiratorisches Hold-Manöver, Referenzphase |
| EH2 | exspiratorisches Hold-Manöver, Testphase |
| G1 | Gewöhnungsphase, Referenzphase |
| G2 | Gewöhnungsphase, Testphase |
| IH1 | inspiratorisches Hold-Manöver, Referenzphase |
| IH2 | inspiratorisches Hold-Manöver, Testphase |
| M1 | Messphase, Referenzphase |
| M2 | Messphase, Testphase |
| P | Druck |
| PEEP1 | PEEP, Referenzphase |
| PEEP2 | PEEP, Testphase |
| $P_{insp}1$ | inspiratorischer Druck, Referenzphase |
| $P_{insp}2$ | inspiratorischer Druck, Testphase |
| R | Referenzphase |
| T1 | Testphase |
| t | Zeit |

**Patentansprüche**

**1.** System (1) zur Beatmung eines Lebewesens (4), umfassend zumindest ein Beatmungsgerät (2) und zumindest eine EIT-Messeinrichtung (3), wobei das Beatmungsgerät (2) zumindest eine steuerbare Atemgasquelle (21) sowie eine programmierbare Steuereinheit (22) zur Steuerung der Atemgasquelle (21) umfasst und wobei die EIT-Messeinrichtung (3) zumindest eine Sensorvorrichtung (31) zur Messung von Impedanzwerten zumindest einer Lunge des Lebewesens sowie zumindest eine Berechnungs- und Auswerteeinheit (32) umfasst, wobei das System (1) die über die zumindest eine Sensorvorrichtung (31) gemessenen Impedanzwerte Pixeln n zuordnet, **dadurch gekennzeichnet, dass** die Steuereinheit (22) dazu eingerichtet ist,

a. in einer Referenzphase (R) der Atemgasquelle (21) Beatmungseinstellungen vorzugeben, wobei zu zumindest einem Zeitpunkt der Referenzphase (R) Impedanzwerte aufgenommen werden und aus den aufgenommenen Impedanzwerten für jedes Pixel n eine erste Kennzahl $C_{R,n}$ berechnet wird und

b. in zumindest einer an die Referenzphase (R) anschließenden Testphase (T1) der Atemgasquelle (21) Beatmungseinstellungen vorzugeben, wobei sich die Beatmungseinstellungen in zumindest einer Beatmungseinstellung von den Beatmungseinstellungen der Referenzphase (R) unterscheiden und wobei zu zumindest einem Zeitpunkt der Testphase (T1) Impedanzwerte aufgenommen werden und aus den aufgenommenen Impedanzwerten für jedes Pixel n eine zweite Kennzahl $C_{T1,n}$ berechnet wird, wobei

sowohl die Referenzphase (R) als auch jede Testphase (T1, Tm) zumindest zwei Atemzüge umfasst, wobei die Referenzphase (R) und jede Testphase (T1, Tm) in zumindest zwei Unterphasen unterteilt werden können, wobei zumindest eine Unterphase eine Gewöhnungsphase (G1, G2) darstellt und zumindest eine Unterphase eine Messphase (M1, M2) darstellt.

2. System nach zumindest einem der vorhergehenden Ansprüche, **dadurch gekennzeichnet, dass** an die zumindest eine Testphase (T1) zumindest eine weitere Testphase (Tm) anschließt, wobei die Beatmungseinstellung jeder weiteren Testphase (Tm) sich von der ersten Testphase (T1) und der Referenzphase (R) in zumindest einer Beatmungseinstellung unterscheidet und wobei das System (1) zu zumindest einem Zeitpunkt jeder weiteren Testphase (Tm) Impedanzwerte aufnimmt und aus den aufgenommenen Impedanzwerten für jedes Pixel n eine zweite Kennzahl $C_{Tm,n}$ berechnet.

3. System (1) nach zumindest einem der vorhergehenden Ansprüche, **dadurch gekennzeichnet, dass** bei jedem Atemzug während der Exspirationsphase ein positiver end-exspiratorischer Druck (PEEP) appliziert wird und während der Inspirationsphase ein um den Wert $\Delta P$ höherer inspiratorischer Druck $P_{insp}$ oder um einen einstellbaren Faktor höheres Tidalvolumens (VT) appliziert wird.

4. System (1) nach zumindest einem der vorhergehenden Ansprüche, **dadurch gekennzeichnet, dass** zum Ende zumindest einer Exspirationsphase ein endexspiratorisches Hold-Manöver (EH1, EH2) durchgeführt wird, wobei das endexspiratorische Hold-Manöver (EH1. EH2) ein Halten des PEEP umfasst, wobei zum Ende zumindest einer Inspirationsphase ein endinspiratorisches Hold-Manöver (IH1, IH2) durchgeführt wird, wobei das endinspiratorische Hold-Manöver (IH1, IH2) ein Halten des inspiratorischen Druckes $P_{insp}$ umfasst.

5. System (1) nach zumindest einem der vorhergehenden Ansprüche, **dadurch gekennzeichnet, dass** das endexspiratorische Hold-Manöver (EH1, EH2) und das endinspiratorische Hold-Manöver (IH1, IH2) während der Messphase (M1, M2) durchgeführt werden, wobei die Messphase (M1, M2) zumindest zwei Atemzüge umfasst und wobei während der Exspirationsphase des vorletzten Atemzugs das endexspiratorische Hold-Manöver (EH1, EH2) durchgeführt wird und wobei während der Inspirationsphase des letzten Atemzuges das endinspiratorische Hold-Manöver (IH1, IH2) durchgeführt wird.

6. System (1) nach zumindest einem der vorhergehenden Ansprüche, **dadurch gekennzeichnet, dass** sowohl während der Referenzphase (R) als auch während der Testphase (T1) zu zumindest zwei Zeitpunkten Impedanzwerte aufgenommen und Pixeln n zugeordnet werden, wobei der erste Zeitpunkt während des endexspiratorischen Hold-Manövers (IH1, IH2) liegt und die aufgenommenen Impedanzwerte den Pixeln n als $I_{Min,n}$ zugeordnet werden und wobei der zweite Zeitpunkt während des endinspiratorischen Hold-Manövers (EH1, EH2) liegt und die aufgenommenen Impedanzwerteden Pixeln n als $I_{Max,n}$ zugeordnet werden, wobei jeweils für die Referenzphase (R) und Testphase (T1) aus den jeweiligen Werten $I_{Min,n}$ und $I_{Max,n}$ folgende Werte berechnet werden:

    a. $\Delta I_n$ für jedes Pixel n als Differenz aus $I_{Max,n}$ und $I_{Min,n}$,
    b. $I_{Min,Sum}$ als Summe aller $I_{Min,n}$,
    c. $I_{Max,Sum}$ als Summer aller $I_{Max,n}$,
    d. $\Delta I_{global}$ als Differenz von $I_{Max,Sum}$ und $I_{Min,Sum}$,

wobei die Berechnung für das jeweilige Pixel nur dann durchgeführt wird und das Pixel nur dann in die Summen $I_{Max,Sum}$ und $I_{Min,Sum}$ einbezogen wird, wenn für das Pixel gilt $\Delta I_n > 0$.

7. System (1) nach zumindest einem der vorherigen Ansprüche, **dadurch gekennzeichnet, dass** die jeweiligen Kennzahlen $C_{R,n}$ und $C_{T1,n}$ der Referenz- bzw. Testphase jeweils aus dem Verhältnis von $\Delta I_n$ zu $\Delta I_{global}$ berechnet werden, wobei

    a. das Verhältnis $\Delta I_n$ zu $\Delta I_{global}$ der Referenzphase mit einem Faktor $C_{R,Stat}$ multipliziert wird um $C_{R,n}$ zu berechnen und
    b. das Verhältnis $\Delta I_n$ zu $\Delta I_{global}$ der Testphase mit einem Faktor $C_{T1,Stat}$ multipliziert wird um $C_{T1,n}$ zu berechnen,

wobei $C_{R,Stat}$ und $C_{T1,Stat}$ aus der Druckdifferenz des endexspiratorischen Hold-Manövers und des endinspiratorischen Hold-Manövers sowie dem Exspirationsvolumen des Atemzuges, welcher dem Atemzug währenddessen das endexspiratorische Hold-Manöver durchgeführt wird, vorrausgeht, bestimmt werden.

8. System (1) nach zumindest einem der vorhergehenden Ansprüche, **dadurch gekennzeichnet, dass** die Kennzahl $C_{T1,n}$ jedes Pixels n mit der Kennzahl $C_{R,n}$ des gleichen Pixels n verglichen wird und das Pixel n einer von zumindest zwei Zahlengruppen zugeordnet wird, wobei

   a. die Pixel n für die gilt, dass $C_{T1,n}$ größer ist als $C_{R,n}$ einer ersten Zahlengruppe WIN zugeordnet werden und
   b. die Pixel n, für die gilt, dass $C_{T1,n}$ kleiner ist als $C_{R,n}$ einer zweiten Zahlengruppe LOSS zugeordnet werden.

9. System (1) nach zumindest einem der vorhergehenden Ansprüche, **dadurch gekennzeichnet, dass** für jedes Pixel n der jeweiligen Zahlengruppe aus den Kennzahlen $C_{T1,n}$ und $C_{R,n}$ eine Kennzahl berechnet wird, wobei

   a. für die Pixel n aus der Zahlengruppe WIN eine Kennzahl $C_{WIN,n}$ berechnet wird und
   b. für die Pixel n aus der Zahlengruppe LOSS eine Kennzahl $C_{LOSS,n}$ berechnet wird und
   c. für die Zahlengruppe WIN mit der Summe der Kennzahlen $C_{WIN,n}$ eine Kennzahl $C_{WIN,global}$ berechnet wird und
   d. für die Zahlengruppe LOSS mit der Summe der Kennzahlen $C_{LOSS,n}$ eine Kennzahl $C_{LOSS,global}$ berechnet wird.

10. System (1) nach zumindest einem der vorhergehenden Ansprüche, **dadurch gekennzeichnet, dass** das System (1) dazu eingerichtet ist, die Kennzahlen $C_{WIN,global}$ und $C_{LOSS,global}$ zu interpretieren und die Größe der Kennzahlen alphanumerisch und/oder graphisch auszugeben, wobei bei der Interpretation der Kennzahlen $C_{WIN,global}$ und $C_{LOSS,global}$ die Beatmungseinstellung einbezogen werden, in welcher sich die Referenzphase (R) und die Testphase (T1) unterscheiden.

11. System (1) nach zumindest einem der vorhergehenden Ansprüche, **dadurch gekennzeichnet, dass** anhand der Interpretation der Kennzahlen $C_{WIN,global}$ und $C_{LOSS,global}$ eine Meldung generiert wird, wobei die Meldung zumindest eine Empfehlung bezüglich der Beatmungseinstellungen enthält und wobei die Meldung alphanumerisch und/oder graphisch ausgegeben wird, wobei die Beatmungseinstellung, zu der die Meldung zumindest eine Empfehlung enthält, zumindest eines von $\Delta P$ und/oder PEEP ist.

12. System (1) nach zumindest einem der vorhergehenden Ansprüche, **dadurch gekennzeichnet, dass** bei einer PEEP-Einstellung, welche in der Testphase (T1) gegenüber der Referenzphase (R) erhöht ist, und

   a. wenn $C_{WIN,global}$ und $C_{LOSS,global}$ unter dem eingestellten Schwellenwert liegen, die Empfehlung ausgegeben wird, die Beatmungseinstellungen nicht zu verändern,
   b. wenn $C_{WIN,global}$ über dem eingestellten Schwellenwert liegt und $C_{LOSS,global}$ unter dem eingestellten Schwellenwert liegt, die Empfehlung ausgegeben wird, den PEEP zu erhöhen,
   c. wenn $C_{WIN,global}$ unter dem eingestellten Schwellenwert liegt und $C_{LOSS,global}$ über dem eingestellten Schwellenwert liegt, die Empfehlung ausgegeben wird, die den PEEP nicht zu verändern und eine Reduzierung vom inspiratorischen Druck $P_{insp}$ und/oder dem exspiratorischen Tidalvolumen VT zu erwägen, wobei zunächst eine Testphase mit reduziertem inspiratorischen Druck $P_{insp}$ eingestellt werden soll,
   d. wenn $C_{WIN,global}$ und $C_{LOSS,global}$ über dem eingestellten Schwellenwert liegen, die Empfehlung ausgegeben wird, den PEEP zu erhöhen und eine Reduzierung vom inspiratorischen Druck $P_{insp}$ und/oder exspiratorischen Tidalvolumen VT zu erwägen, wobei zunächst eine Testphase mit reduziertem inspiratorischen Druck $P_{insp}$ eingestellt werden soll,
   und/oder bei einer PEEP-Einstellung, welche in der Testphase (T1) gegenüber der Referenzphase (R) reduziert ist
   e. wenn $C_{WIN,global}$ und $C_{LOSS,global}$ unter dem eingestellten Schwellenwert liegen, die Empfehlung ausgegeben wird, die Beatmungseinstellungen nicht zu verändern und/oder den PEEP zu reduzieren,
   f. wenn $C_{WIN,global}$ über dem eingestellten Schwellenwert liegt und $C_{LOSS,global}$ unter dem eingestellten Schwellenwert liegt, die Empfehlung ausgegeben wird, den PEEP zu reduzieren,
   g. wenn $C_{WIN,global}$ unter dem eingestellten Schwellenwert liegt und $C_{LOSS,global}$ über dem eingestellten Schwellenwert liegt, die Empfehlung ausgegeben wird, die Einstellung des PEEP nicht zu verändern und/oder zu erhöhen und ein Rekrutierungsmanöver zu erwägen,
   h. wenn $C_{WIN,global}$ und $C_{LOSS,global}$ über dem eingestellten Schwellenwert liegen, die Empfehlung ausgegeben wird, die Einstellung des PEEP nicht zu verändern und ein Rekrutierungsmanöver zu erwägen sowie, soweit möglich, $P_{insp}$ und/oder VT zu reduzieren.

13. System (1) nach zumindest einem der vorhergehenden Ansprüche, **dadurch gekennzeichnet, dass** bei einer $P_{insp}$- und/oder VT-Einstellung, welche in der Testphase (T1) gegenüber der Referenzphase (R) erhöht ist und

a. wenn $C_{WIN,global}$ und $C_{LOSS,global}$ unter dem eingestellten Schwellenwert liegen, die Empfehlung ausgegeben wird, die Beatmungseinstellungen nicht zu verändern

b. wenn $C_{WIN,global}$ über dem eingestellten Schwellenwert liegt und $C_{LOSS,global}$ unter dem eingestellten Schwellenwert liegt, die Empfehlung ausgegeben wird, den PEEP zu erhöhen

c. wenn $C_{WIN,global}$ unter dem eingestellten Schwellenwert liegt und $C_{LOSS,global}$ über dem eingestellten Schwellenwert liegt, die Empfehlung ausgegeben wird, die Einstellung des PEEP und/oder des $P_{insp}$ und/oder von VT zu reduzieren

d. wenn $C_{WIN,global}$ und $C_{LOSS,global}$ über dem eingestellten Schwellenwert liegen, die Empfehlung ausgegeben wird, den PEEP zu erhöhen und/oder, soweit möglich, den $P_{insp}$ und/oder das Tidalvolumen zu reduzieren.

und/oder bei einer $P_{insp}$- und/oder VT-Einstellung, welche in der Testphase (T1) gegenüber der Referenzphase (R) reduziert ist und

e. wenn $C_{WIN,global}$ und $C_{LOSS,global}$ unter dem eingestellten Schwellenwert liegen, die Empfehlung ausgegeben wird, die Beatmungseinstellungen nicht zu verändern,

f. wenn $C_{WIN,global}$ über dem eingestellten Schwellenwert liegt und $C_{LOSS,global}$ unter dem eingestellten Schwellenwert liegt, die Empfehlung ausgegeben wird, den PEEP und/oder $P_{insp}$ und/oder VT zu reduzieren,

g. wenn $C_{WIN,global}$ unter dem eingestellten Schwellenwert liegt und $C_{LOSS,global}$ über dem eingestellten Schwellenwert liegt, die Empfehlung ausgegeben wird, den PEEP zu erhöhen,

h. wenn $C_{WIN,global}$ und $C_{LOSS,global}$ über dem eingestellten Schwellenwert liegen, die Empfehlung ausgegeben wird, den PEEP zu erhöhen und, soweit möglich, den $P_{insp}$ und/oder das Tidalvolumen zu reduzieren.

**14.** System (1) nach zumindest einem der vorhergehenden Ansprüche, **dadurch gekennzeichnet, dass** anhand der Interpretation der Kennzahlen $C_{WIN,global}$ und $C_{LOSS,global}$ ein Befund erstellt wird und die Meldung neben der Empfehlung auch zumindest einen Befund enthält.

**15.** System (1) nach zumindest einem der vorhergehenden Ansprüche, **dadurch gekennzeichnet, dass** die Steuereinrichtung (22) dazu ausgebildet ist, die ausgegebenen Empfehlungen automatisch umzusetzen und entsprechende Beatmungseinstellungen vorzunehmen.

**16.** System (1) nach zumindest einem der vorhergehenden Ansprüche, **dadurch gekennzeichnet, dass** das System (1) dazu eingerichtet ist folgende Verfahrensschritte durchzuführen

a. Gewöhnen (101) des Lebewesens (4) an Beatmungseinstellungen einer Referenzphase R
b. Messen (102) von Impedanzen der Lunge des Lebewesens (4) während der Referenzphase R
c. Gewöhnen (103) des Lebewesens (4) an Beatmungseinstellungen einer Testphase T1
d. Messen (104) von Impedanzen der Lunge des Lebewesens (4) während der Testphase T1
e. Berechnen (105, 106) von Kennzahlen $C_{R,n}$ und $C_{T1,n}$ aus den Impedanzwerten aus dem Schritt des Messens (102, 104)
f. Vergleichen (107) der Kennzahlen $C_{R,n}$ und $C_{T1,n}$
g. Einordnen (108) der Kennzahlen $C_{R,n}$ und $C_{T1,n}$ in Zahlengruppen WIN und LOSS
h. Berechnen (109, 110) von Kennzahlen $C_{WIN,global}$ und $C_{LOSS,global}$
i. Vergleichen (111, 112) der Kennzahlen $C_{WIN,global}$ und $C_{LOSS,global}$ mit Schwellenwerten
j. Auswerten (113), wobei das Auswerten (113) zumindest die Generierung einer graphischen Darstellung der Kennzahlen $C_{WIN,global}$ und $C_{LOSS,global}$ umfasst und wobei der Schritt des Auswertens (113) optional die Erstellung eines Befundes unter Berücksichtigung der Kennzahlen $C_{WIN,global}$ und $C_{LOSS,global}$ umfasst
k. Empfehlen (114), wobei das Empfehlen (114) zumindest die Generierung einer alphanumerischen Ausgabe von Empfehlungen bezüglich der Beatmungseinstellungen umfasst.

**Claims**

**1.** A system (1) for ventilating an organism (4), comprising at least one ventilator (2) and at least one EIT measuring apparatus (3), wherein the ventilator (2) comprises at least one controllable breathing gas source (21) and a programmable control unit (22) for controlling the breathing gas source (21) and wherein the EIT measuring apparatus (3) comprises at least one sensor device (31) for measuring impedance values of at least one lung of the organism as well as at least one calculation and evaluation unit (32), wherein the system (1) assigns the impedance values measured using the at least one sensor device (31) to pixels n, **characterized in that** the control unit (22) is configured

a. to specify ventilation settings for the breathing gas source (21) in a reference phase (R), wherein impedance values are recorded at at least one point in time in the reference phase (R) and a first characteristic number $C_{R,n}$ is calculated for each pixel n from the recorded impedance values, and

b. to specify ventilation settings for the breathing gas source (21) in at least one test phase (T1) that follows on from the reference phase (R), wherein the ventilation settings differ from the ventilation settings of the reference phase (R) in at least one ventilation setting and wherein impedance values are recorded at at least one point in time in the test phase (T1) and a second characteristic number $C_{T1,n}$ is calculated for each pixel n from the recorded impedance values, wherein both the reference phase (R) and each test phase (T1, Tm) comprises at least two breaths, wherein the reference phase (R) and each test phase (T1, Tm) can be divided into at least two subphases, wherein at least one subphase constitutes an acclimatization phase (G1, G2) and at least one subphase constitutes a measurement phase (M1, M2).

2. The system according to at least one of the preceding claims, **characterized in that** at least one further test phase (Tm) follows on from the at least one test phase (T1), wherein the ventilation setting of each further test phase (Tm) differs from the first test phase (T1) and the reference phase (R) in at least one ventilation setting and wherein the system (1) records impedance values at at least one point in time in each further test phase (Tm) and calculates a second characteristic number $C_{Tm,n}$ for each pixel n from the recorded impedance values.

3. The system (1) according to at least one of the preceding claims, **characterized in that** a positive end-expiratory pressure (PEEP) is applied during each breath during the expiration phase and an inspiratory pressure $P_{insp}$ that is higher by the value $\Delta P$ or a tidal volume (VT) that is higher by a settable factor is applied during the inspiration phase.

4. The system (1) according to at least one of the preceding claims, **characterized in that** an end-expiratory hold maneuver (EH1, EH2) is performed at the end of at least one expiration phase, wherein the end-expiratory hold maneuver (EH1, EH2) comprises holding the PEEP, wherein an end-inspiratory hold maneuver (IH1, IH2) is performed at the end of at least one inspiration phase, wherein the end-inspiratory hold maneuver (IH1, IH2) comprises holding the inspiratory pressure $P_{insp}$.

5. The system (1) according to at least one of the preceding claims, **characterized in that** the end-expiratory hold maneuver (EH1, EH2) and the end-inspiratory hold maneuver (IH1, IH2) are performed during the measurement phase (M1, M2), wherein the measurement phase (M1, M2) comprises at least two breaths and wherein the end-expiratory hold maneuver (EH1, EH2) is performed during the expiration phase of the penultimate breath and wherein the end-inspiratory hold maneuver (IH1, IH2) is performed during the inspiration phase of the last breath.

6. The system (1) according to at least one of the preceding claims, **characterized in that** impedance values are recorded and assigned to pixels n both during the reference phase (R) and during the test phase (T1) at at least two points in time, wherein the first point in time is during the end-expiratory hold maneuver (IH1, IH2) and the recorded impedance values are assigned to the pixels n as $I_{Min,n}$ and wherein the second point in time is during the end-inspiratory hold maneuver (EH1, EH2) and the recorded impedance values are assigned to the pixels n as $I_{Max,n}$, wherein the following values are calculated from the respective values $I_{Min,n}$ and $I_{Max,n}$ in each case for the reference phase (R) and test phase (T1):

a. $\Delta I_n$ for each pixel n as the difference between $I_{Max,n}$ and $I_{Min,n}$,
b. $I_{Min,Sum}$ as the sum of all $I_{Min,n}$,
c. $I_{Max,Sum}$ as the sum of all $I_{Max,n}$,
d. $\Delta I_{global}$ as the difference between $I_{Max,Sum}$ and $I_{Min,Sum}$,

wherein the calculation is only performed for the relevant pixel and the pixel is only included in the sums $I_{Max,Sum}$ and $I_{Min,Sum}$ if $\Delta I_n > 0$ applies for the pixel.

7. The system (1) according to at least one of the preceding claims, **characterized in that** the respective characteristic numbers $C_{R,n}$ and $C_{T1,n}$ of the reference phase and test phase, respectively, are calculated in each case from the ratio of $\Delta I_n$ to $\Delta I_{global}$, wherein

a. the ratio of $\Delta I_n$ to $\Delta I_{global}$ of the reference phase is multiplied by a factor $C_{R,Stat}$ in order to calculate $C_{R,n}$, and
b. the ratio of $\Delta I_n$ to $\Delta I_{global}$ of the test phase is multiplied by a factor $C_{T1,Stat}$ in order to calculate $C_{T1,n}$,

wherein $C_{R,Stat}$ and $C_{T1,Stat}$ are determined from the pressure difference between the end-expiratory hold maneuver and the end-inspiratory hold maneuver as well as from the expiration volume of the breath preceding the breath during which the end-expiratory hold maneuver is performed.

8. The system (1) according to at least one of the preceding claims, **characterized in that** the characteristic number $C_{T1,n}$ of each pixel n is compared with the characteristic number $C_{R,n}$ of the same pixel n and the pixel n is assigned to one of at least two groups of numbers, wherein

   a. the pixels n for which $C_{T1,n}$ is greater than $C_{R,n}$ are assigned to a first group of numbers WIN, and
   b. the pixels n for which $C_{T1,n}$ is less than $C_{R,n}$ are assigned to a second group of numbers LOSS.

9. The system (1) according to at least one of the preceding claims, **characterized in that** a characteristic number is calculated from the characteristic numbers $C_{T1,n}$ and $C_{R,n}$ for each pixel n of the relevant group of numbers, wherein

   a. a characteristic number $C_{WIN,n}$ is calculated for the pixels n from the group of numbers WIN, and
   b. a characteristic number $C_{LOSS,n}$ is calculated for the pixels n from the group of numbers LOSS, and
   c. a characteristic number $C_{WIN,global}$ is calculated for the group of numbers WIN using the sum of the characteristic numbers $C_{WIN,n}$, and
   d. a characteristic number $C_{LOSS,global}$ is calculated for the group of numbers LOSS using the sum of the characteristic numbers $C_{LOSS,n}$.

10. The system (1) according to at least one of the preceding claims, **characterized in that** the system (1) is configured to interpret the characteristic numbers $C_{WIN,global}$ and $C_{LOSS,global}$ and to alphanumerically and/or graphically output the magnitude of the characteristic numbers, wherein the ventilation setting in which the reference phase (R) and the test phase (T1) differ from one another is included in the interpretation of the characteristic numbers $C_{WIN,global}$ and $C_{LOSS,global}$.

11. The system (1) according to at least one of the preceding claims, **characterized in that** a report is generated based on the interpretation of the characteristic numbers $C_{WIN,global}$ and $C_{LOSS,global}$, wherein the report contains at least one recommendation with regard to the ventilation settings and wherein the report is output alphanumerically and/or graphically, wherein the ventilation setting for which the report contains at least one recommendation is at least one of ΔP and/or PEEP.

12. The system (1) according to at least one of the preceding claims, **characterized in that,** in the case of a PEEP setting that is increased in the test phase (T 1) relative to the reference phase (R) and

   a. if $C_{WIN,global}$ and $C_{LOSS,global}$ are below the set threshold value, the recommendation not to change the ventilation settings is output,
   b. if $C_{WIN,global}$ is above the set threshold value and $C_{LOSS,global}$ is below the set threshold value, the recommendation to increase the PEEP is output,
   c. if $C_{WIN,global}$ is below the set threshold value and $C_{LOSS,global}$ is above the set threshold value, the recommendation not to change the PEEP and to consider reducing the inspiratory pressure $P_{insp}$ and/or the expiratory tidal volume VT is output, wherein a test phase with a reduced inspiratory pressure $P_{insp}$ should firstly be set,
   d. if $C_{WIN,global}$ and $C_{LOSS,global}$ are above the set threshold value, the recommendation to increase the PEEP and to consider reducing the inspiratory pressure $P_{insp}$ and/or expiratory tidal volume VT is output, wherein a test phase with a reduced inspiratory pressure $P_{insp}$ should firstly be set,
   and/or in the case of a PEEP setting that is reduced in the test phase (T1) relative to the reference phase (R),
   e. if $C_{WIN,global}$ and $C_{LOSS,global}$ are below the set threshold value, the recommendation not to change the ventilation settings and/or to reduce the PEEP is output,
   f. if $C_{WIN,global}$ is above the set threshold value and $C_{LOSS,global}$ is below the set threshold value, the recommendation to reduce the PEEP is output,
   g. if $C_{WIN,global}$ is below the set threshold value and $C_{LOSS,global}$ is above the set threshold value, the recommendation not to change the PEEP setting and/or to increase same and to consider a recruitment maneuver is output,
   h. if $C_{WIN,global}$ and $C_{LOSS,global}$ are above the set threshold value, the recommendation not to change the PEEP setting and to consider a recruitment maneuver and, if possible, to reduce the $P_{insp}$ and/or VT is output.

13. The system (1) according to at least one of the preceding claims, **characterized in that,** in the case of a $P_{insp}$ and/or VT setting that is increased in the test phase (T1) relative to the reference phase (R) and

a. if $C_{WIN,global}$ and $C_{LOSS,global}$ are below the set threshold value, the recommendation not to change the ventilation settings is output,

b. if $C_{WIN,global}$ is above the set threshold value and $C_{LOSS,global}$ is below the set threshold value, the recommendation to increase the PEEP is output,

c. if $C_{WIN,global}$ is below the set threshold value and $C_{LOSS,global}$ is above the set threshold value, the recommendation to reduce the PEEP and/or $P_{insp}$ and/or VT setting is output,

d. if $C_{WIN,global}$ and $C_{LOSS,global}$ are above the set threshold value, the recommendation to increase the PEEP and/or, if possible, to reduce the $P_{insp}$ and/or the tidal volume is output,

and/or in the case of a $P_{insp}$ and/or VT setting that is reduced in the test phase (T1) relative to the reference phase (R) and

e. if $C_{WIN,global}$ and $C_{LOSS,global}$ are below the set threshold value, the recommendation not to change the ventilation settings is output,

f. if $C_{WIN,global}$ is above the set threshold value and $C_{LOSS,global}$ is below the set threshold value, the recommendation to reduce the PEEP and/or $P_{insp}$ and/or VT is output,

g. if $C_{WIN,global}$ is below the set threshold value and $C_{LOSS,global}$ is above the set threshold value, the recommendation to increase the PEEP is output,

h. if $C_{WIN,global}$ and $C_{LOSS,global}$ are above the set threshold value, the recommendation to increase the PEEP and, if possible, to reduce the $P_{insp}$ and/or the tidal volume is output.

14. The system (1) according to at least one of the preceding claims, **characterized in that** a finding is created based on the interpretation of the characteristic numbers $C_{WIN,global}$ and $C_{LOSS,global}$ and the report also contains at least one finding in addition to the recommendation.

15. The system (1) according to at least one of the preceding claims, **characterized in that** the control apparatus (22) is designed to automatically implement the output recommendations and to apply corresponding ventilation settings.

16. The system (1) according to at least one of the preceding claims, **characterized in that** the system (1) is configured to carry out the following method steps:

a. acclimatizing (101) the organism (4) to ventilation settings of a reference phase R,

b. measuring (102) impedances of the lung of the organism (4) during the reference phase R,

c. acclimatizing (103) the organism (4) to ventilation settings of a test phase T1,

d. measuring (104) impedances of the lung of the organism (4) during the test phase T1,

e. calculating (105, 106) characteristic numbers $C_{R,n}$ and $C_{T1,n}$ from the impedance values from the step of measuring (102, 104),

f. comparing (107) the characteristic numbers $C_{R,n}$ and $C_{T1,n}$,

g. classifying (108) the characteristic numbers $C_{R,n}$ and $C_{T1,n}$ into groups of numbers WIN and LOSS,

h. calculating (109, 110) characteristic numbers $C_{WIN,global}$ and $C_{LOSS,global}$,

i. comparing (111, 112) the characteristic numbers $C_{WIN,global}$ and $C_{LOSS,global}$ with threshold values,

j. evaluating (113), wherein the evaluating (113) at least comprises generating a graphical representation of the characteristic numbers $C_{WIN,global}$ and $C_{LOSS,global}$ and wherein the step of evaluating (113) optionally comprises creating a finding taking into account the characteristic numbers $C_{WIN,global}$ and $C_{LOSS,global}$,

k. recommending (114), wherein the recommending (114) at least comprises generating an alphanumerical output of recommendations with regard to the ventilation settings.

## Revendications

1. Système (1) pour la ventilation d'un être vivant (4), comportant au moins un ventilateur (2) et au moins un appareil de mesure EIT (3), dans lequel l'appareil de ventilation (2) comporte au moins une source de gaz respiratoire commandable (21) et une unité de commande programmable (22) pour la commande de la source de gaz respiratoire (21) et dans lequel l'appareil de mesure EIT (3) comporte au moins un dispositif de capteur (31) pour la mesure de valeurs d'impédance d'au moins un poumon de l'être vivant et au moins une unité de calcul et d'évaluation (32), dans lequel le système (1) attribue les valeurs d'impédance mesurées par le biais de l'au moins un dispositif de capteur (31) à des pixels n, **caractérisé en ce que** l'unité de commande (22) est configurée pour

a. spécifier des réglages de ventilation à la source de gaz respiratoire (21) dans une phase de référence (R), dans lequel des valeurs d'impédance sont enregistrées à au moins un moment de la phase de référence (R) et un premier nombre caractéristique $C_{R,n}$ est calculé pour chaque pixel n à partir des valeurs d'impédance enregistrées et

b. au cours d'au moins une phase de test (T1) de la consécutive à la phase de référence (R), spécifier des réglages de ventilation à la source de gaz respiratoire (21), dans lequel les réglages de ventilation se distinguent des réglages de ventilation de la phase de référence (R) au moins dans un réglage de ventilation et dans lequel des valeurs d'impédance sont enregistrées à au moins un moment de la phase de test (T1) et un deuxième nombre caractéristique $C_{T1,n}$ est calculé pour chaque pixel n à partir des valeurs d'impédance enregistrées, dans lequel

aussi bien la phase de référence (R) que chaque phase de test (T1, Tm) comportent au moins deux respirations, dans lequel la phase de référence (R) et chaque phase de test (T1, Tm) peuvent être divisées en au moins deux sous-phases, dans lequel au moins une sous-phase représente une phase d'habituation (G1, G2) et au moins une sous-phase représente une phase de mesure (M1, M2).

2. Système selon l'une au moins des revendications précédentes, **caractérisé en ce qu'**au moins une phase de test supplémentaire (Tm) fait suite à l'au moins une phase de test (T1), dans lequel le réglage de ventilation de chaque phase de test supplémentaire (Tm) se distingue de la première phase de test (T1) et de la phase de référence (R) dans au moins un réglage de ventilation et dans lequel le système (1) enregistre des valeurs d'impédance à au moins un moment de chaque phase de test supplémentaire (Tm) et calcule un deuxième nombre caractéristique $C_{Tm,n}$ pour chaque pixel n à partir des valeurs d'impédance enregistrées.

3. Système (1) selon l'une au moins des revendications précédentes, **caractérisé en ce que** lors de chaque respiration, une pression expiratoire finale positive (PEP) est appliquée pendant la phase d'expiration et une pression inspiratoire $P_{insp}$ plus élevée selon la valeur $\Delta P$ ou un volume tidal (VT) plus élevé selon un facteur réglable est appliqué pendant la phase d'inspiration.

4. Système (1) selon l'une au moins des revendications précédentes, **caractérisé en ce qu'**une manoeuvre de maintien expiratoire finale (EH1, EH2) est effectuée à la fin d'au moins une phase d'expiration, dans lequel la manoeuvre de maintien expiratoire finale (EH1, EH2) comporte un maintien de la PEP, dans lequel une manoeuvre de maintien inspiratoire finale (IH1, IH2) est effectuée à la fin d'au moins une phase d'inspiration, dans lequel la manoeuvre de maintien inspiratoire finale (IH1, IH2) comporte un maintien de la pression inspiratoire $P_{insp}$.

5. Système (1) selon l'une au moins des revendications précédentes, **caractérisé en ce que** la manoeuvre de maintien expiratoire finale (EH1, EH2) et la manoeuvre de maintien inspiratoire finale (IH1, IH2) sont effectuées pendant la phase de mesure (M1, M2), dans lequel la phase de mesure (M1, M2) comporte au moins deux respirations et dans lequel la manoeuvre de maintien expiratoire finale (EH1, EH2) est effectuée pendant la phase d'expiration de l'avant-dernière respiration et dans lequel la manoeuvre de maintien inspiratoire finale (IH1, IH2) est effectuée pendant la phase d'inspiration de la dernière respiration.

6. Système (1) selon l'une au moins des revendications précédentes, **caractérisé en ce que** des valeurs d'impédance sont enregistrées et attribuées à des pixels n à au moins deux moments aussi bien pendant la phase de référence (R) que pendant la phase de test (T1), dans lequel le premier moment se situe pendant la manoeuvre de maintien expiratoire finale (IH1, IH2) et les valeurs d'impédance enregistrées sont attribuées aux pixels n en tant que $I_{Min,n}$ et dans lequel le deuxième moment se situe pendant la manoeuvre de maintien inspiratoire finale (EH1, EH2) et les valeurs d'impédance enregistrées sont attribuées aux pixels n en tant que $I_{Max,n}$, dans lequel, pour la phase de référence (R) et la phase de test (T1) respectivement, les valeurs suivantes sont calculées à partir des valeurs $I_{Min,n}$ et $I_{Max,n}$ respectives :

a. $\Delta I_n$ pour chaque pixel n en tant que différence entre $I_{Max,n}$ et $I_{Min,n}$,
b. $I_{min,Sum}$ en tant que somme de toutes les $I_{Min,n}$,
c. $I_{Max,Sum}$ en tant que somme de toutes les $I_{Max,n}$,
d. $\Delta I_{global}$ en tant que différence de $I_{Max,Sum}$ et $I_{min,Sum}$,

dans lequel le calcul pour le pixel respectif est effectué seulement si et le pixel est ajouté aux sommes $I_{Max,Sum}$ et $I_{min,Sum}$ seulement si $\Delta I_n > 0$ est appliqué au pixel.

7. Système (1) selon l'une au moins des revendications précédentes, **caractérisé en ce que** les nombres caractéristiques $C_{R,n}$ et $C_{T1,n}$ respectifs de la phase de référence ou de test sont calculés respectivement à partir du rapport

de $\Delta I_n$ à $\Delta I_{global}$, dans lequel

    a. le rapport de $\Delta I_n$ à $\Delta I_{global}$ de la phase de référence est multiplié par un facteur $C_{R,Stat}$ afin de calculer $C_{R,n}$ et
    b. le rapport de $\Delta I_n$ à $\Delta I_{global}$ de la phase de test est multiplié par un facteur $C_{T1,Stat}$ afin de calculer $C_{T1,n}$,

dans lequel $C_{R,Stat}$ et $C_{T1,Stat}$ sont déterminés à partir de la différence de pression de la manoeuvre de maintien expiratoire finale et de la manoeuvre de maintien inspiratoire finale ainsi que du volume d'expiration de la respiration précédant la respiration pendant laquelle la manoeuvre de maintien expiratoire finale est effectuée.

8. Système (1) selon l'une au moins des revendications précédentes, **caractérisé en ce que** le nombre caractéristique $C_{T1,n}$ de chaque pixel n est comparé avec le nombre caractéristique $C_{R,n}$ du même pixel n et le pixel n est attribué à l'un parmi au moins deux groupes de nombres, dans lequel

    a. les pixels pour lesquels s'applique que $C_{T1,n}$ est supérieur à $C_{R,n}$ sont attribués à un premier groupe de nombres WIN et
    b. les pixels pour lesquels s'applique que $C_{T1,n}$ est inférieur à $C_{R,n}$ sont attribués à un deuxième groupe de nombres LOSS.

9. Système (1) selon l'une au moins des revendications précédentes, **caractérisé en ce qu'**un nombre caractéristique est calculé à partir des nombres caractéristiques $C_{T1,n}$ et $C_{R,n}$ pour chaque pixel n du groupe de nombres respectif, dans lequel

    a. un nombre caractéristique $C_{WIN,n}$ est calculé pour les pixels issus du groupe de nombres WIN et
    b. un nombre caractéristique $C_{LOSS,n}$ est calculé pour les pixels issus du groupe de nombres LOSS et
    c. un nombre caractéristique $C_{WIN,global}$ est calculé pour le groupe de nombres WIN avec la somme des nombres caractéristiques $C_{WIN,n}$ et
    d. un nombre caractéristique $C_{LOSS,global}$ est calculé pour le groupe de nombres LOSS avec la somme des nombres caractéristiques $C_{LOSS,n}$.

10. Système (1) selon l'une au moins des revendications précédentes, **caractérisé en ce que** le système (1) est configuré pour interpréter les nombres caractéristiques $C_{WIN,global}$ et $C_{LOSS,global}$ et pour émettre la grandeur des nombres caractéristiques sous forme alphanumérique et/ou graphique, dans lequel le réglage de ventilation dans lequel la phase de référence (R) et la phase de test (T1) se distinguent est intégré dans de l'interprétation des nombres caractéristiques $C_{WIN,global}$ et $C_{LOSS,global}$.

11. Système (1) selon l'une au moins des revendications précédentes, **caractérisé en ce qu'**un message est généré sur la base de l'interprétation des nombres caractéristiques $C_{WIN,global}$ et $C_{LOSS,global}$, dans lequel le message contient au moins une recommandation concernant les réglages de ventilation et dans lequel le message est émis sous forme alphanumérique et/ou graphique, dans lequel le réglage de ventilation pour lequel le message contient au moins une recommandation est l'un au moins parmi $\Delta P$ et/ou PEP.

12. Système (1) selon l'une au moins des revendications précédentes, **caractérisé en ce que** lors d'un réglage PEP augmenté dans la phase de test (T1) par rapport à la phase de référence (R), et

    a. lorsque $C_{WIN,global}$ et $C_{LOSS,global}$ sont en dessous de la valeur seuil réglée, la recommandation émise consiste à ne pas modifier les réglages de ventilation,
    b. lorsque $C_{WIN,global}$ est au-dessus de la valeur seuil réglée et que $C_{LOSS,global}$ est en dessous de la valeur seuil réglée, la recommandation émise consiste à augmenter la PEP,
    c. lorsque $C_{WIN,global}$ est en dessous de la valeur seuil réglée et que $C_{LOSS,global}$ est au-dessus de la valeur seuil réglée, la recommandation émise consiste à ne pas modifier la PEP et à envisager une réduction de la pression inspiratoire $P_{insp}$ et/ou du volume tidal expiratoire VT, sachant qu'une phase de test avec une pression inspiratoire $P_{insp}$ réduite doit tout d'abord être réglée,
    d. lorsque $C_{WIN,global}$ et $C_{LOSS,global}$ sont au-dessus de la valeur seuil réglée, la recommandation émise consiste à augmenter la PEP et à envisager une réduction de la pression inspiratoire $P_{insp}$ et/ou du volume tidal expiratoire VT, sachant qu'une phase de test avec une pression inspiratoire $P_{insp}$ réduite doit tout d'abord être réglée, et/ou lors d'un réglage PEP réduit dans la phase de test (T1) par rapport à la phase de référence (R),
    e. lorsque $C_{WIN,global}$ et $C_{LOSS,global}$ sont en dessous de la valeur seuil réglée, la recommandation émise consiste à ne pas modifier les réglages de ventilation et/ou à réduire la PEP,

f. lorsque $C_{WIN,global}$ est au-dessus de la valeur seuil réglée et que $C_{LOSS,global}$ est en dessous de la valeur seuil réglée, la recommandation émise consiste à réduire la PEP,

g. lorsque $C_{WIN,global}$ est en dessous de la valeur seuil réglée et que $C_{LOSS,global}$ est au-dessus de la valeur seuil réglée, la recommandation émise consiste à ne pas modifier et/ou à augmenter le réglage de la PEP et à envisager une manoeuvre de recrutement,

h. lorsque $C_{WIN,global}$ et $C_{LOSS,global}$ sont au-dessus de la valeur seuil réglée, la recommandation émise consiste à ne pas modifier le réglage de la PEP et à envisager une manoeuvre de recrutement, et, dans la mesure du possible, à réduire $P_{insp}$ et/ou VT.

13. Système (1) selon l'une au moins des revendications précédentes, **caractérisé en ce que** lors d'un réglage $P_{insp}$ et/ou VT augmenté dans la phase de test (T1) par rapport à la phase de référence (R) et

a. lorsque $C_{WIN,global}$ et $C_{LOSS,global}$ sont en dessous de la valeur seuil réglée, la recommandation émise consiste à ne pas modifier les réglages de ventilation,

b. lorsque $C_{WIN,global}$ est au-dessus de la valeur seuil réglée et que $C_{LOSS,global}$ est en dessous de la valeur seuil réglée, la recommandation émise consiste à augmenter la PEP,

c. lorsque $C_{WIN,global}$ est en dessous de la valeur seuil réglée et que $C_{LOSS,global}$ est au-dessus de la valeur seuil réglée, la recommandation émise consiste à réduire le réglage de la PEP et/ou de la $P_{insp}$ et/ou du VT

d. lorsque $C_{WIN,global}$ et $C_{LOSS,global}$ sont au-dessus de la valeur seuil réglée, la recommandation émise consiste à augmenter la PEP et/ou, dans la mesure du possible, à réduire la $P_{insp}$ et/ou le volume tidal,

et/ou lors d'un réglage $P_{insp}$ et/ou VT réduit dans la phase de test (T1) par rapport à la phase de référence (R) et

e. lorsque $C_{WIN,global}$ et $C_{LOSS,global}$ sont en dessous de la valeur seuil réglée, la recommandation émise consiste à ne pas modifier les réglages de ventilation,

f. lorsque $C_{WIN,global}$ est au-dessus de la valeur seuil réglée et que $C_{LOSS,global}$ est en dessous de la valeur seuil réglée, la recommandation émise consiste à réduire la PEP et/ou $P_{insp}$ et/ou VT,

g. lorsque $C_{WIN,global}$ est en dessous de la valeur seuil réglée et que $C_{LOSS,global}$ est au-dessus de la valeur seuil réglée, la recommandation émise consiste à augmenter la PEP,

h. lorsque $C_{WIN,global}$ et $C_{LOSS,global}$ sont au-dessus de la valeur seuil réglée, la recommandation émise consiste à augmenter la PEP et, dans la mesure du possible, à réduire la $P_{insp}$ et/ou le volume tidal.

14. Système (1) selon l'une au moins des revendications précédentes, **caractérisé en ce qu'**un rapport est établi sur la base de l'interprétation des nombres caractéristiques $C_{WIN,global}$ et $C_{LOSS,global}$ et le message contient également au moins un rapport en plus de la recommandation.

15. Système (1) selon l'une au moins des revendications précédentes, **caractérisé en ce que** l'appareil de commande (22) est conçu pour convertir automatiquement les recommandations émises et pour effectuer des réglages de ventilation correspondants.

16. Système (1) selon l'une au moins des revendications précédentes, **caractérisé en ce que** le système (1) est configuré pour mettre en oeuvre les étapes de procédé suivantes

a. habituation (101) de l'être vivant (4) à des réglages de ventilation d'une phase de référence R

b. mesure (102) d'impédances du poumon de l'être vivant (4) pendant la phase de référence R

c. habituation (103) de l'être vivant (4) aux réglages de ventilation d'une phase de test T1

d. mesure (104) d'impédances du poumon de l'être vivant (4) pendant la phase de test T1

e. calcul (105, 106) de nombres caractéristiques $C_{R,n}$ et $C_{T1,n}$ à partir des valeurs d'impédance provenant de l'étape de mesure (102, 104)

f. comparaison (107) des nombres caractéristiques $C_{R,n}$ et $C_{T1,n}$

g. classement (108) des nombres caractéristiques $C_{R,n}$ et $C_{T1,n}$ en groupe de nombres WIN et LOSS

h. calcul (109, 110) de nombres caractéristiques $C_{WIN,global}$ et $C_{LOSS,global}$

i. comparaison (111, 112) des nombres caractéristiques $C_{WIN,global}$ et $C_{LOSS,global}$ avec des valeurs seuil

j. évaluation (113), l'évaluation (113) comportant au moins la génération d'une représentation graphique des nombres caractéristiques $C_{WIN,global}$ et $C_{LOSS,global}$ et l'étape de l'évaluation (113) comportant facultativement l'établissement d'un rapport tenant compte des nombres caractéristiques $C_{WIN,global}$ et $C_{LOSS,global}$

k. recommandation (114), la recommandation (114) comportant au moins la génération d'une émission alphanumérique de recommandations concernant les réglages de ventilation.

**Figur 1**

**Figur 2**

**Figur 3**

Figur 4

**Figur 5**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102013203177 A1
- DE 102017007224 A1

**In der Beschreibung aufgeführte Nicht-Patentliteratur**

- **LIONHEART, W.R.B.** *Physiological Measurement*, 2004, vol. 25, 1
- **SCHULLCKE, B.** *Scientific Reports*, 2016, vol. 6, 25951
- **COSTA, E.L.** *Intensive Care Med*, 2009, vol. 35, 1132-1137